# EUROPEAN PATENT APPLICATION

(11) **EP 4 174 472 A1**
(43) Date of publication of application: **03.05.2023**
(21) Application number: 20942188.2
(22) Date of filing: 25.06.2020
(51) Int. Cl.: G01N 3/32

(54) **MATERIAL EVALUATION DEVICE, MATERIAL EVALUATION METHOD, AND MATERIAL EVALUATION PROGRAM**

(71) Applicant: FUJITSU LIMITED, Kawasaki-shi, Kanagawa 211-8588 (JP)
(72) Inventor: SOEDA, Takeshi, Kawasaki-shi, Kanagawa 211-8588 (JP)
(74) Representative: Hoffmann Eitle
(86) International application number: PCT/JP2020/025020
(87) International publication number: WO 2021/260883

(57) **Abstract**

To efficiently acquire physical property values of materials.

In a storage unit (10) of a material evaluation device 1, N (N is an integer equal to or greater than 2) hysteresis curves (40) each indicating a change in a physical quantity (Q) of each time with respect to a change in a physical quantity (P) of N times at a certain coordinate (a) of a material (30) is stored. For example, the physical quantity (P) is temperature, and the physical quantity (Q) is a deformation amount. A processing unit (20) refers to the storage unit (10), scans each of the N hysteresis curves at the coordinate (a) with a value of the predetermined physical quantity (Q) to extract a point, one-dimensionally arrays the extracted points to generate one-dimensional information (41) regarding the physical quantity (Q), and calculates the physical property values of the material (30) using the one-dimensional information (41). For example, a time when a mutation point occurs is obtained from the pieces of one-dimensional information (41) corresponding to N times, and linear and nonlinear physical property values of the material (30) are calculated based on transition of the physical quantity (Q) between the time at or before the mutation point and the time at or after the mutation point.

## Description

### FIELD

The present invention relates to a material evaluation device, a material evaluation method, and a material evaluation program.

### BACKGROUND

There are known technologies of predicting a behavior that occurs when a thermal cycle is repeated, of a multilayer substrate in which a metal layer including one of Cu, Al, a Cu alloy, or an Al alloy, and an insulating layer including insulating ceramics are laminated, by a simulation method using a hardening rule. Furthermore, there is also a known technology of creating material constants to be introduced into the hardening rule based on a curve that reflects a relationship between stress and strain of a metal layer when saturated, in which the metal layer hardens and has increased stress due to repeated deformation.

### CITATION LIST

### PATENT DOCUMENT

Patent Document 1: Japanese Laid-open Patent Publication No. 2011-203042

### SUMMARY

### [TECHNICAL PROBLEM]

By the way, as one of methods of evaluating materials, for example, electronic devices such as large scale integration (LSI) packages, there is a method of repeatedly applying a load such as temperature to materials to evaluate durability of the materials. With this method, the number of man-hours needed to repeatedly apply a load becomes enormous, and the evaluation may become inefficient.

To cope with the problem, it is conceivable to construct a model that reproduces the materials on a computer using a computer aided engineering (CAE), predict a state of the materials when the load is applied using the model, and improve the efficiency of the evaluation.

Physical property values of the materials are used to construct such a model. However, there may be cases where the number and combinations of elements that constitute the materials are enormous, and effective physical property values of the elements within the materials are not easy to become clear. Therefore, it takes a huge amount of calculation and time to acquire the physical property values that can be used in the model that reproduces the materials, and it may be difficult to efficiently acquire the physical property values of the materials. As a result, it may occur that the model that reproduces the materials may not be able to be efficiently constructed.

In one aspect, an object of the present invention is to efficiently acquire physical property values of materials.

### [SOLUTION TO PROBLEM]

In one aspect, provided is a material evaluation device including: a storage unit configured to store N (N is an integer equal to or greater than 2) hysteresis curves each representing a change in a second physical quantity of each time with respect to a change in a first physical quantity of N times at at least one of a plurality of positions of a material; and a processing unit configured to refer to the storage unit, scan each of the N hysteresis curves with a value of the predetermined second physical quantity, for at least one of the plurality of positions, to extract a point, one-dimensionally array the extracted points to generate one-dimensional information regarding the second physical quantity, and calculate a physical property value of the material using the generated one-dimensional information regarding the second physical quantity.

Furthermore, in another aspect, a material evaluation method and a material evaluation program are provided.

According to an aspect of the embodiments, a material evaluation device includes a storage unit configured to store N (N is an integer equal to or greater than 2) hysteresis curves that each represent a change in a second physical quantity of each time with respect to a change in a first physical quantity of N times at at least one of a plurality of positions of a material; and a processing unit configured to refer to the storage unit, scan each of the N hysteresis curves with a value of the predetermined second physical quantity, for at least one of the plurality of positions, to extract a point, and one-dimensionally array the extracted points to generate one-dimensional information regarding the second physical quantity, and calculate a physical property value of the material using the generated one-dimensional information regarding the second physical quantity.

### [ADVANTAGEOUS EFFECTS OF INVENTION]

In one aspect, it becomes possible to efficiently obtain physical property values of materials.

Objects, features, and advantages of the present invention will become apparent from the following description in conjunction with the accompanying drawings, which illustrate preferable embodiments of the present invention.

### BRIEF DESCRIPTION OF DRAWINGS

FIG. 1A and FIG. 1B are views for describing an example of a TC test;
FIG. 2 is a diagram for describing deformation of an LSI package during the TC test;
FIG. 3 is a diagram for describing hysteresis curves according to a first embodiment;
FIG. 4 is a diagram for describing an example of a material evaluation device according to the first embodiment;
FIG. 5 is a diagram for describing an example of a material evaluation system according to a second embodiment;
FIG. 6 is a diagram for describing an example of a hardware configuration of a material evaluation device according to the second embodiment;
FIG. 7 is a diagram for describing an example of functions that the material evaluation device has, according to the second embodiment;
FIG. 8 is a view for describing an example of an FEM model according to the second embodiment;
FIG. 9A, FIG. 9B, and FIG. 9C are diagrams for describing an example of processing of a deformation amount one-dimensional information generation unit according to the second embodiment;
FIG. 10A and FIG. 10B are diagrams for describing an example of processing of a deformation amount to TC count two-dimensional information generation unit according to the second embodiment;
FIG. 11A and FIG. 11B are diagrams for describing an example of deformation amount to TC count two-dimensional information according to the second embodiment;
FIG. 12A, FIG. 12B, and FIG. 12C are diagrams for describing a hardening coefficient and an acceleration coefficient obtained from the deformation amount to TC count two-dimensional information according to the second embodiment;
FIG. 13A and FIG. 13B are diagrams for describing an example of processing of a TC count one-dimensional information generation unit according to the second embodiment;
FIG. 14 is a diagram for describing an example of processing of a TC count to coordinate two-dimensional information generation unit according to the second embodiment;
FIG. 15A and FIG. 15B are diagrams for describing an example of processing of a calculation unit according to the second embodiment;
FIG. 16A and FIG. 16B are diagrams illustrating a calculation example of physical property values according to the second embodiment;
FIG. 17 is a flowchart illustrating an example of a mutation point determination processing flow according to the second embodiment;
FIG. 18 is a flowchart illustrating an example of a TC count prediction processing flow according to the second embodiment;
FIG. 19 is a flowchart illustrating an example of a physical property value calculation processing flow according to the second embodiment; and
FIG. 20 is a flowchart illustrating an example of a TC test monitoring processing flow according to the second embodiment.

### DESCRIPTION OF EMBODIMENTS

There are a wide variety of tests for evaluating reliability of electronic devices such as LSI packages. One of the tests is a temperature cycle (TC) test. In the TC test, an electronic device, which is a material to be evaluated, is continuously exposed to a certain temperature range, and its mechanical durability is evaluated.

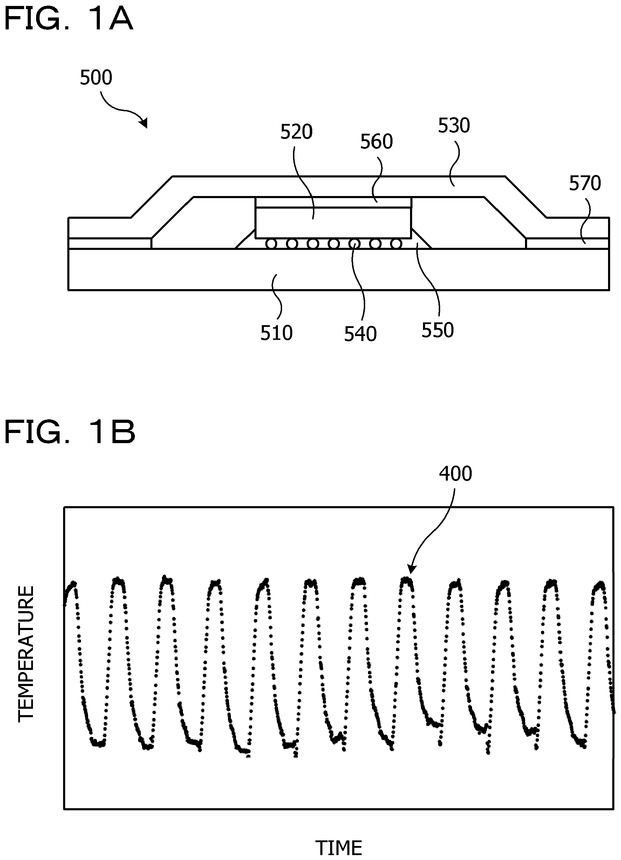

FIG. 1A and FIG. 1B are views for describing an example of the TC test. FIG. 1A is a view schematically illustrating a cross section of an essential part of an example of the electronic device, and FIG. 1B is a view schematically illustrating an example of a temperature load in the TC test.

FIG. 1A illustrates an LSI package 500 as an example of the electronic device that is the material to be evaluated by the TC test. The LSI package 500 illustrated in FIG. 1A and FIG. 1B are includes a substrate 510, a semiconductor chip 520, and a lid 530. The substrate 510 is a circuit board such as a printed circuit board, and includes an insulating layer and wiring layers provided on a surface of the insulating layer and inside thereof although not illustrated here. A semiconductor chip 520 is mounted on such a substrate 510 using bumps 540 such as solder. For example, an underfill (UF) 550 is filled between the substrate 510 and the semiconductor chip 520. The lid 530 is provided to cover the substrate 510 and the semiconductor chip 520 mounted thereon. A thermal interface material (TIM) 560 is provided between the lid 530 and an upper surface of the semiconductor chip 520, for example. An edge of the lid 530 and the substrate 510 are bonded using an adhesive 570, for example.

During the TC test, a temperature load in which one cycle is set within a predetermined temperature range, such as a temperature cycle 400 illustrated in FIG. 1B, is repeatedly applied to the LSI package 500 illustrated in FIG. 1A, for example. Although the temperature range varies depending on conditions under which the LSI package 500 is used, or the like, the temperature range is set to a range between a stress-free film formation temperature, for example, 175°C, and an extreme temperature, for example, -55°C.

In general, in TC tests, electrical characteristics, such as electrical resistance values, are monitored. When the durability of the LSI package 500 reaches its limit due to the temperature load, the LSI package 500 is physically destroyed, and an electrical resistance value changes due to disconnection of wiring paths inside the LSI package 500 and the like. However, the electrical resistance value is not necessarily the best indicator for monitoring. The electrical resistance value is useful as an indicator for capturing occurrence of destruction accompanied by relatively large deformation. Meanwhile, since the LSI package 500 has a huge number of wiring paths, even if relatively small deformation or local deformation occurs due to the temperature load, a change amount in the electrical resistance value is small and sometimes falls within a range of measurement error. In actual measurements, in 100 to 1000 times of TC tests, there are some cases where the electrical resistance value suddenly changes in last one cycle, or at most in last two or three cycles.

To improve such a relatively inefficient TC test, capturing mechanical deformation of the LSI package 500 instead of the electrical resistance value has been attempted.

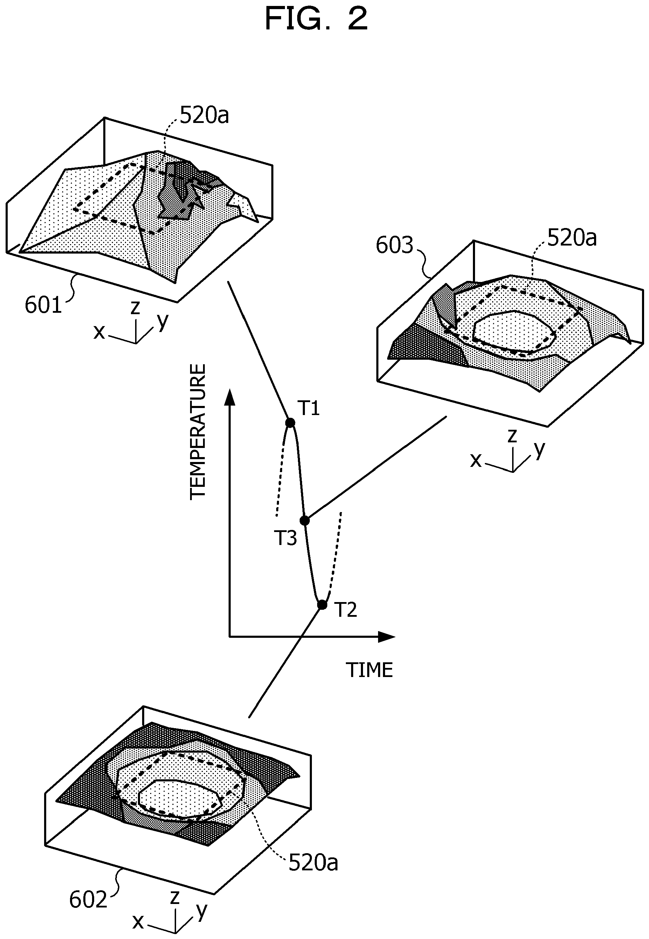

FIG. 2 is a diagram for describing deformation of the LSI package during the TC test.

FIG. 2 schematically illustrates a material image 601, a material image 602, and a material image 603, which are images of the LSI package 500 respectively captured at an upper limit temperature T1, a lower limit temperature T2, and their intermediate temperature T3, of the one-cycle temperature load applied to the LSI package 500 in the TC test. A position 520a of the semiconductor chip 520 is illustrated by the dotted line in each of the material image 601, the material image 602, and the material image 603 of FIG. 2.

As illustrated in FIG. 2, the LSI package 500 can undergo various mechanical deformations at various locations during the one cycle from the upper limit temperature T1 to the lower limit temperature T2 of the temperature load during the TC test. If such mechanical deformations that occur in the LSI package 500 can be analyzed, it will become possible to clarify a mechanism leading to fracture and factors directly related to the durability.

A model that reproduces the LSI package 500 on a computer is effective in capturing the mechanical deformation of the LSI package 500. By using such a model, it becomes possible to reproduce changes in the LSI package 500 and its raw materials caused by the temperature changes, and to predict fine and local changes before destruction.

CAE is used to construct the model of the LSI package 500. A finite element method (FEM) in the CAE category is effective for evaluating electrical, mechanical, or magnetic material properties. In the material evaluation by the FEM, physical property values of the raw materials forming the LSI package 500 are used. For example, in the case of mechanical characteristics, physical property values such as linear expansion coefficient, Young's modulus, and Poisson's ratio are used.

Although the size and layout of the raw materials forming the LSI package 500 can be easily obtained from computer aided design (CAD) data and microscopic images, it is often difficult to obtain physical property values of the raw materials. For example, the LSI package 500 is composed of a large number of raw materials, and in many cases, the effective physical property values of the raw materials are unknown due to contamination of impurities during a manufacturing process and fluctuations in thermal history. Depending on the raw material, the physical property values are not published, and the values are sometimes unknown in the first place. Furthermore, the deformation that occurs in the LSI package 500 is determined by a combination of the raw materials, and thus can depend on its size and layout. Moreover, when the deformation of the LSI package 500 reaches an irreversible plastic deformation area, the physical property values of the raw materials can change at each temperature load cycle count (TC count), it is sometimes difficult to know how the physical property values affect the deformation.

Here, as an example of a method of constructing the model, a technology as illustrated in Document 1 below has been proposed.

Document 1 Advanced Metallization Conference 2019 (ADMETA Plus 2019, "Advanced Physical Modelling Method to the LSI Package Deformation with the HOG Image Feature")

In the technology of Document 1, a physical property value is obtained by collating a measurement result of a deformation amount when a load is applied with a calculation result of a deformation amount when the physical property value is used as a variable. To improve the efficiency of this collation, it is devised to reduce an information amount by extracting histograms of oriented gradients (HOG) feature. However, in a case of an LSI package with an asymmetric design structure, local deformations occur simultaneously and various gradients occur at various locations. Therefore, it may be difficult to reduce the information amount as a result of the need to execute many feature calculations in order to capture orientation and direction of the gradients of the deformations. If the information amount is reduced by reducing the number of features, the accuracy of the model will decrease. If the accuracy of the model is tried to improve, the number of features will increase and time cost will increase.

In view of the above points, materials to be evaluated are analyzed and physical property values are calculated using a method to be described below as an embodiment.

[First Embodiment]

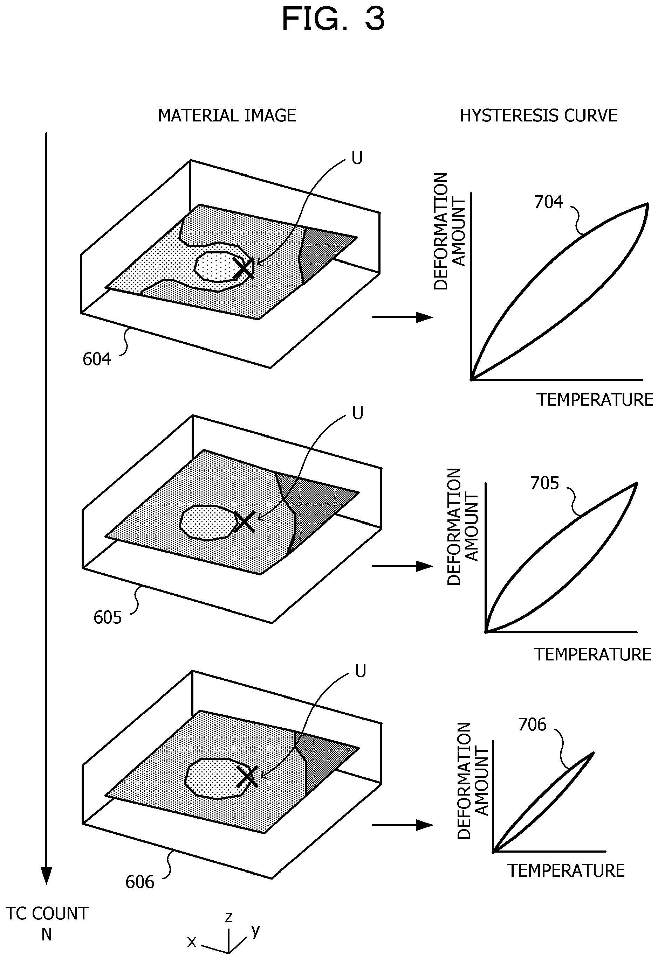

FIG. 3 is a diagram for describing hysteresis curves according to a first embodiment.

In a TC test, a material such as an LSI package expands or contracts in proportion to a linear expansion coefficient when a load temperature is changed. The linear expansion coefficient varies depending on a raw material (component) included in the material. Furthermore, a deformation of the material also depends on a Young's modulus (difficulty in deformation) and a Poisson's ratio (strain aspect ratio) of the raw material. Therefore, the material such as the LSI package, which includes a plurality of raw materials, may undergo various deformations at various locations depending on a combination of the raw materials and a temperature applied.

In the TC test, for each TC count, a material image in a temperature range of one cycle or a material image at a predetermined temperature within the temperature range is captured and acquired using an imaging device such as a camera. FIG. 3 schematically illustrates a material image 604, a material image 605, and a material image 606 acquired at certain same temperature within the temperature range of one cycle, corresponding to the TC count N of three, and arranged in an order of the TC count N from top to bottom of the drawing. The material can be variously deformed in the temperature range of one cycle, as illustrated in FIG. 2 above. Moreover, the material may also exhibit different deformation behaviors at the same temperature with different TC counts N, as illustrated in FIG. 3.

Focusing on the deformation amount at a predetermined coordinate U of the material, deformations represented by a two-dimensional hysteresis curve 704, a hysteresis curve 705, and a hysteresis curve 706 as illustrated in FIG. 3 can occur during the temperature load of one cycle, in other words, at respective counts of the TC count. The hysteresis curve 704, the hysteresis curve 705, and the hysteresis curve 706 represent transitions of the deformation amount (vertical axis) of the material with respect to the temperature (horizontal axis) during the temperature load of one cycle. In general, stress and strain are considered to be proportional within an elastic limit of the material. In a precise sense, paths are slightly different between a loading process and an unloading process, and are not linear and draw a loop as illustrated in FIG. 3. This phenomenon is called elastic hysteresis, and it is a phenomenon that can be detected by precise measurement. When the elastic limit is greatly exceeded and unloading and reloading are repeated in a plastic area, a more distinct loop is formed. This phenomenon is called plastic hysteresis. In the TC test, an elastic deformation is dominant at high temperatures close to stress-free, and a plastic deformation tends to be promoted at lower temperatures.

Here, the mechanical deformation of the material is analyzed using the hysteresis curve obtained from the material image acquired during the TC test, as illustrated in FIG. 3, a physical property value of the material is calculated based on the analysis, and the model of the material is constructed.

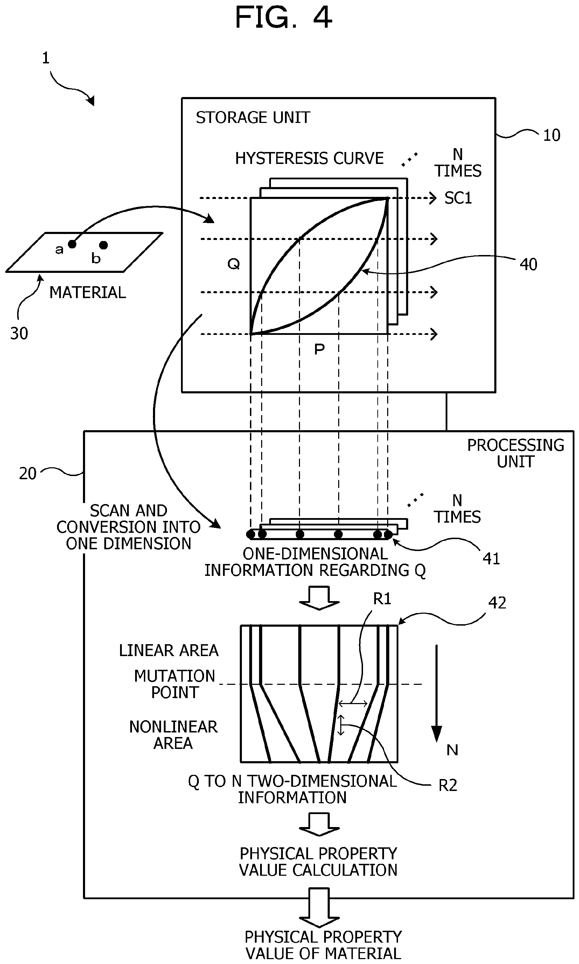

FIG. 4 is a diagram for describing an example of a material evaluation device according to the first embodiment.

A material evaluation device 1 illustrated in FIG. 4 includes a storage unit 10 and a processing unit 20. The storage unit 10 may be a volatile semiconductor memory such as a random access memory (RAM) or may be a non-volatile storage such as a hard disk drive (HDD) or a flash memory. The processing unit 20 is, for example, a processor such as a central processing unit (CPU) or a digital signal processor (DSP). The processing unit 20 may include an electronic circuit for a specific application such as an application specific integrated circuit (ASIC), a field programmable gate array (FPGA), and the like. The processor executes a program stored in a memory such as a RAM (which may be the storage unit 10).

The storage unit 10 stores a hysteresis curve 40 obtained from a material image acquired during the TC test of a material 30. The storage unit 10 stores the hysteresis curve 40 representing a deformation behavior at certain coordinate a obtained from a plurality of material images acquired at each count, for the each count of N (N is an integer equal to or greater than 2) TC tests corresponding to the temperature load cycle count (TC count) for the material 30. In other words, the storage unit 10 stores N hysteresis curves 40 corresponding to the TC count N for the certain coordinate a of the material 30. The storage unit 10 may store N hysteresis curves 40 corresponding to the TC count N for coordinate b different from the coordinate a of the material 30, and may store N hysteresis curves 40 corresponding to the TC count N for each of all of coordinates of the material 30.

Note that the coordinate of the material 30 is information indicating a position of each mesh when the material 30 is divided into meshes with predetermined granularity. The coordinate may be information indicating a point on the material 30, or information indicating an area having a predetermined size on the material 30. The area having a predetermined size on the material 30 may be a two-dimensional plane or a three-dimensional space.

Furthermore, the material 30 may have various shapes, and moreover, may contain a raw material of one type or may include a plurality of raw materials of the same type or different types.

The hysteresis curve 40 stored in the storage unit 10 represents a change amount in a physical quantity Q with respect to a physical quantity P. In the case of this example of the TC test, the hysteresis curve 40 stored in the storage unit 10 represents the deformation amount of the material 30 (for example, its coordinate a) with respect to the temperature during the TC test. The hysteresis curve 40 is two-dimensional information indicating a relationship between the physical quantity P and the physical quantity Q, that is, a relationship between the temperature and the deformation amount of the material 30 in this example.

The processing unit 20 refers to the storage unit 10 and performs processing of converting the hysteresis curve 40 stored in the storage unit 10 into one dimension. For example, the processing unit 20 converts each of the N hysteresis curves 40 corresponding to the TC count N obtained for the coordinate a of the material 30 into one dimension.

When converting each hysteresis curve 40 into one dimension, the processing unit 20 scans the hysteresis curve 40 with a value of the predetermined physical quantity Q, in this example, a value of the deformation amount of the predetermined material 30, as illustrated by four dotted arrows SC1 in FIG. 4, for example, and extracts points on the hysteresis curve 40. The value of the predetermined physical quantity Q for scanning, that is, the number of scans, and an interval between scans (scan resolution) are set to predetermined values. Note that the value of the predetermined physical quantity Q for scanning is the same for scan of each hysteresis curve 40.

In scanning the hysteresis curve 40, points on the hysteresis curve are set to °1", and other points are set to "0". The hysteresis curve 40 is converted into one dimension by superimposing information of "1" and "0" obtained by scanning the hysteresis curve 40 a plurality of times. In the example of FIG. 4, a total of six points are extracted as the information of "1" by four scans (dotted arrows SC1). Such one-dimensional processing is performed for each of the N hysteresis curves 40 corresponding to the TC count N for the coordinate a of the material 30, and one-dimensional information 41 for each hysteresis curve 40, that is, the one-dimensional information 41 regarding the deformation amount at the coordinate a of the material 30 in this example is generated.

Moreover, the processing unit 20 performs processing of analyzing the material 30 and calculating a physical property value thereof, using the generated one-dimensional information 41.

For example, the processing unit 20 arrays, with respect to the TC count N, the one-dimensional information 41 regarding the deformation amount at the coordinate a of the material 30, which has been generated for each of the N hysteresis curves 40 corresponding to the TC count N for the coordinate a of the material 30. Therefore, the processing unit 20 generates two-dimensional information 42 of the deformation amount at the coordinate a of the material 30 with respect to the TC count N, in other words, physical quantity Q to TC count N two-dimensional information 42.

In the two-dimensional information 42, regarding the N hysteresis curves 40 corresponding to the TC count N at the coordinate a of the material 30, if the shapes of the hysteresis curves 40 at a certain count and at the next count of the certain count are the same as each other, transition of the deformation amount (physical quantity Q) at the coordinate a of the material 30 is parallel to a direction of an axis (vertical axis) of the TC count N. If the shapes of the hysteresis curves 40 at the certain count and the next count of the certain count are different from each other, the transition of the deformation amount at the coordinate a of the material 30 is not parallel to the axis direction of the TC count N, and becomes a direction inclined with respect to the axis direction of the TC count N.

The processing unit 20 obtains the TC count at which the deformation amount at the coordinate a of the material 30 in the two-dimensional information 42 begins to transition while being inclined with a predetermined inclination value or larger, the deformation amount having been transitioning in parallel or with less than the predetermined inclination value based on the parallelism to the axis direction of the TC count N. That is, the processing unit 20 obtains a mutation point at which tendency of the transition of the deformation amount at the coordinate a of the material 30 in the two-dimensional information 42 changes.

Since the hysteresis curve 40 does not differ or the difference is relatively small depending on the TC count among the TC counts at or before the mutation point, the deformation at the coordinate a of the material 30, which occurs during the TC test, can be considered to be an elastic deformation or mainly the elastic deformation. Since the hysteresis curve 40 has a relatively large difference depending on the TC count among the TC counts at or after the mutation point, the deformation at the coordinate a of the material 30, which occurs during the TC test, can be considered to be a plastic deformation or mainly the plastic deformation. The mutation point can be considered to represent a boundary between the elastic deformation and the plastic deformation at the coordinate a of the material 30, that is, the TC count at or near a yield point indicating the elastic limit.

At the TC count at or after the mutation point of the plastic deformation or mainly the plastic deformation, destruction due to deformation tends to progress at the coordinate a of the material 30, and a hardening coefficient of a stress-strain curve can nonlinearly change. A change amount R1 in a cross direction in the two-dimensional information 42, in other words, in a direction orthogonal to the axis direction of the TC count N can be regarded as representing a strain accumulation amount, and the hardening coefficient (how much it becomes hard) of the coordinate a of the material 30 can be estimated from the strain accumulation amount. Furthermore, a change amount R2 in a longitudinal direction in the two-dimensional information 42, in other words, in the axis direction of the TC count N can be regarded as representing a strain accumulation speed, and an acceleration coefficient (how much the hardening has progressed) of the coordinate a of the material 30 can be estimated from the strain accumulation speed.

The processing unit 20 calculates the physical property values of the material 30 using the information of the deformation amount included in the material image, which is a measured value of the material 30, and the FEM model set for the material 30. For example, the processing unit 20 compares the measured value of the deformation amount at the coordinate a of the material 30 with a calculated value of the FEM model including the physical property values at the coordinate a of the material 30 as parameters. Then, the processing unit 20 repeats the calculation while changing a combination of the physical property values until the calculated value matches the measured value or a difference from the measured value becomes equal to or less than a predetermined threshold value.

At that time, the processing unit 20 first compares the measured value obtained at the TC count before the mutation point or the measured value obtained at still another TC count obtained from the two-dimensional information 42 with the calculated value of the FEM model including linear physical property values as parameters. Then, the calculation is repeated while changing the combination of the linear physical property values until the calculated value of the FEM model matches the measured value at the TC count at or before the mutation point or the measured value at still another TC, or the difference from the measured value becomes equal to or less than the predetermined threshold value. Here, the linear physical property values are the linear expansion coefficient, Young's modulus, Poisson's ratio, and the like. The processing unit 20 calculates the linear physical property values at the coordinate a of the material 30 through such repeated calculations. In a case where a plurality of raw materials is included at the coordinate a of the material 30, the processing unit 20 calculates the linear physical property values by such repeated calculations for each raw material.

Next, the processing unit 20 compares the measured value obtained at the TC count after the mutation point or the measured value obtained at still another TC count obtained from the two-dimensional information 42 with the calculated value of the FEM model including nonlinear physical property values as parameters. Then, the calculation is repeated while changing the combination of the nonlinear physical property values until the calculated value of the FEM model matches the measured value at the TC count at or after the mutation point or the measured value at still another TC, or the difference from the measured value becomes equal to or less than the predetermined threshold value. Here, the nonlinear physical property value is the hardening coefficient of the stress-strain curve, or the like. The processing unit 20 calculates the nonlinear physical property values at the coordinate a of the material 30 through such repeated calculations. When a plurality of raw materials are included in the coordinate a of the material 30, the processing unit 20 calculates nonlinear physical property values by such repeated calculations for each of the raw materials. Note that, as the hardening coefficient, the strain accumulation amount that appears as the change amount R2 in the two-dimensional information 42 can also be used.

The processing unit 20 outputs the calculated linear physical property value and nonlinear physical property value at the coordinate a of the material 30 to an outside, for example, displays the physical property values on a display device or the like.

In this way, the processing unit 20 generates the one-dimensional information 41 as described above from the N hysteresis curves 40 corresponding to the TC count N for the coordinate a of the material 30, generates the two-dimensional information 42 using the one-dimensional information 41, and calculates the physical property values at the coordinate a of the material 30 further using the two-dimensional information 42. The processing unit 20 may also similarly generate the one-dimensional information 41 and the two-dimensional information 42 from the N hysteresis curves 40 corresponding to the TC count N for the coordinate b different from the coordinate a of the material 30 or each of all of the coordinates of the material 30, and calculate the physical property values of the material 30 using the one-dimensional information 41 and the two-dimensional information 42.

In the material evaluation device 1, the number of combinations of the physical property values and the number of repetitions of the calculation can be reduced as compared with a case of calculating each physical property value by comparing the calculated value of the FEM model including both the linear and nonlinear physical property values as parameters with the measured values corresponding to the TC count N. Furthermore, in the material evaluation device 1, even if there is no stress-strain curve for each raw material and for each temperature in the TC test, the information regarding the strain accumulation amount at a predetermined coordinate can be obtained from the two-dimensional information 42, and an effective hardening coefficient during the TC test can be relatively easily estimated. According to the material evaluation device 1, it becomes possible to efficiently calculate the physical property values of the material 30 and efficiently construct the FEM model that reproduces the material 30 with high accuracy.

### [Second Embodiment]

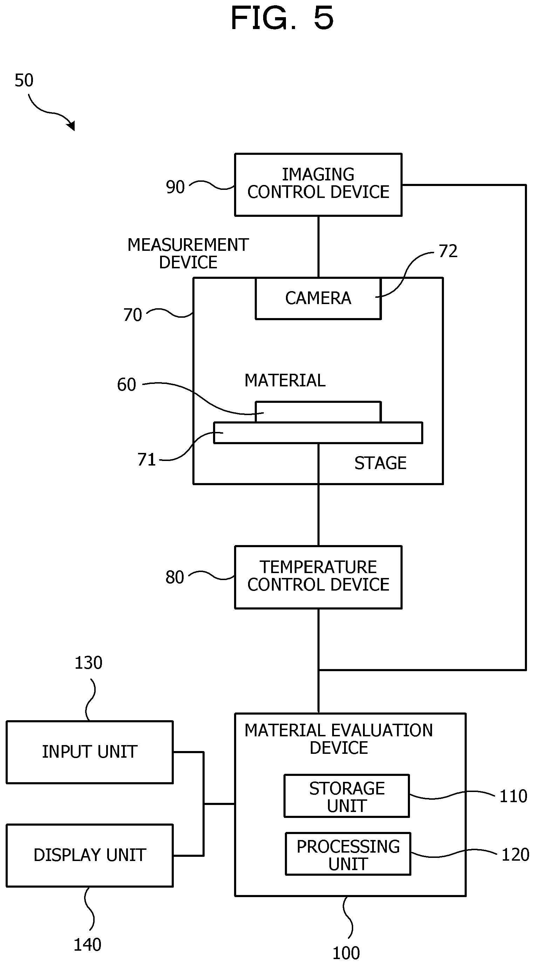

FIG. 5 is a diagram for describing an example of a material evaluation system according to a second embodiment.

A material evaluation system 50 illustrated in FIG. 5 includes a measurement device 70, a temperature control device 80, an imaging control device 90, a material evaluation device 100, an input device 130, and a display device 140.

The measurement device 70 has a stage 71 on which a material 60 to be evaluated is set, and a camera 72 that captures the material 60 set on the stage 71. The temperature of the stage 71 is controlled by the temperature control device 80. During a TC test, the material 60 set on the stage 71 is controlled such that a temperature load in which one cycle is set to a predetermined temperature range is repeatedly given a predetermined number of times (TC count) by the stage 71 in which the temperature is controlled by the temperature control device 80. Capture of the material 60 by the camera 72 is controlled by the imaging control device 90. The imaging control device 90 controls timing to capture the material 60 at a predetermined temperature or the like.

Note that the material 60 may have various shapes, and moreover, may contain a raw material of one type or may include a plurality of raw materials of the same type or different types.

The material evaluation device 100 has a storage unit 110 and a processing unit 120.

The storage unit 110 stores an image of the material 60 captured by the camera 72 and a material image (TC test material image) indicating a deformation amount of the material 60 during the TC test. A TC test material image is obtained by measuring a surface difference of the material 60 before and after deformation by, for example, a digital image correlation method. The storage unit 110 may further store a hysteresis curve generated by the processing unit 120 as will be described below, information generated based thereon, an FEM model to be used for calculating physical property values, calculated physical property values, and the like.

The processing unit 120 performs processing of controlling the temperature control device 80 and the imaging control device 90. The processing unit 120 also performs processing of generating a hysteresis curve indicating the deformation amount of the material 60 during the TC test, using the TC test material image stored in the storage unit 110. The processing unit 120 further performs processing of generating information regarding deformation of the material 60 during the TC test, using the generated hysteresis curve and calculating physical property values of the material 60 based on the generated information.

The input device 130 and the display device 140 are connected to the material evaluation device 100. For example, control conditions for performing predetermined processing by the processing unit 120 are input by the input device 130. The display device 140 outputs, for example, the TC test material image acquired for the material 60, content of the processing performed by the processing unit 120, and the information obtained by the processing performed by the processing unit 120 (calculated physical property values, and the like).

Next, a hardware configuration of the material evaluation device 100 will be described.

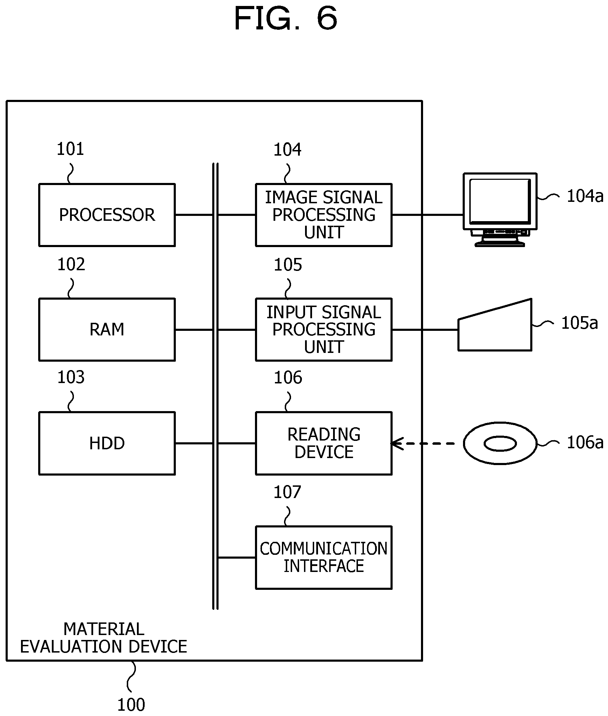

FIG. 6 is a diagram for describing an example of a hardware configuration of the material evaluation device according to the second embodiment.

The material evaluation device 100 has a processor 101, a RAM 102, an HDD 103, an image signal processing unit 104, an input signal processing unit 105, a reading device 106, and a communication interface 107.

The processor 101 may be a multiprocessor. The processor 101 is, for example, a CPU, a DSP, an ASIC, an FPGA, or the like. The processor 101 may be a combination of two or more types of the CPU, DSP, ASIC, FPGA, and the like.

The RAM 102 is a main storage device of the material evaluation device 100. The RAM 102 temporarily stores at least part of an operating system (OS) program or an application program to be executed by the processor 101. Furthermore, the RAM 102 stores various types of data used for processing by the processor 101.

The HDD 103 is an auxiliary storage device of the material evaluation device 100. The HDD 103 magnetically writes data to and reads data from a built-in magnetic disk. The HDD 103 stores OS programs, application programs, and various types of data. The material evaluation device 100 may include another type of auxiliary storage device such as a solid state drive (SSD), or may include a plurality of auxiliary storage devices.

The image signal processing unit 104 causes a display 104a connected to the material evaluation device 100 to display an image according to an instruction from the processor 101. As the display 104a, a CRT display, a liquid crystal display, or the like can be used.

The input signal processing unit 105 acquires an input signal from an input device 105a connected to the material evaluation device 100 and outputs the input signal to the processor 101. As the input device 105a, for example, a pointing device such as a mouse or a touch panel, a keyboard, or the like can be used.

The reading device 106 is a device that reads a program and data recorded on a recording medium 106a. As the recording medium 106a, a magnetic disk such as a flexible disk (FD) or an HDD, an optical disk such as a compact disc (CD) or a digital versatile disc (DVD), or a magneto-optical disk (MO) can be used, for example. Furthermore, a non-volatile semiconductor memory such as a flash memory card can also be used as the recording medium 106a, for example. The reading device 106 stores programs and data read from the recording medium 106a in the RAM 102 or HDD 103 according to an instruction from the processor 101, for example.

The communication interface 107 is an interface for the material evaluation device 100 to communicate with other devices via a network.

Next, functions that the material evaluation device 100 has will be described.

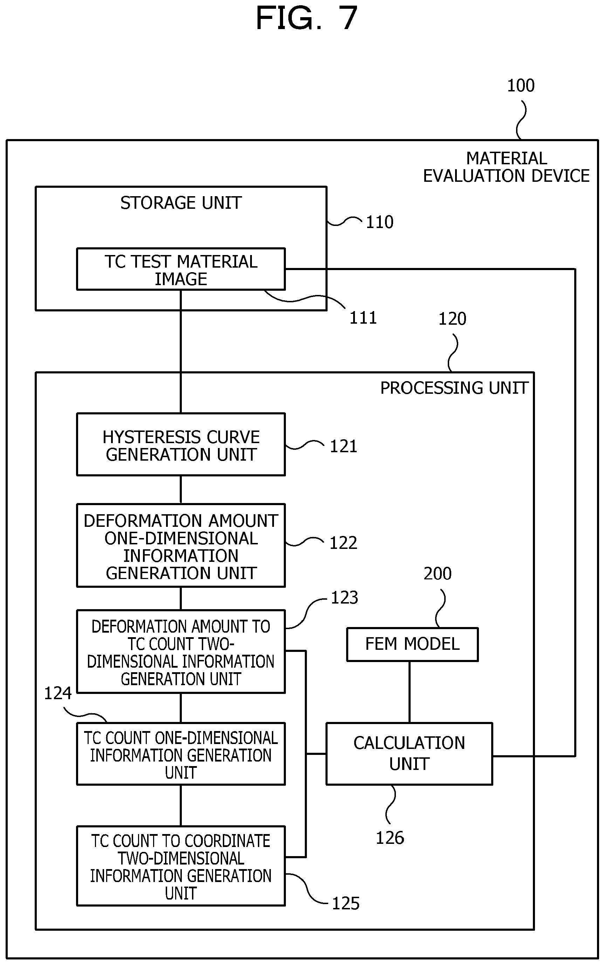

FIG. 7 is a diagram for describing an example of functions that the material evaluation device has, according to the second embodiment.

The material evaluation device 100 illustrated in FIG. 7 includes the storage unit 110 and the processing unit 120. The processing unit 120 includes a hysteresis curve generation unit 121, a deformation amount one-dimensional information generation unit 122, a deformation amount to TC count two-dimensional information generation unit 123, a TC count one-dimensional information generation unit 124, a TC count to coordinate two-dimensional information generation unit 125, and a calculation unit 126.

The hysteresis curve generation unit 121 generates a hysteresis curve (hysteresis curve 150 to be described below) indicating transition of the deformation amount with respect to the temperature of the material 60, using a TC test material image 111 acquired for the material 60 during the TC test and stored in the storage unit 110. The hysteresis curve generation unit 121 generates a hysteresis curve indicating transition of the deformation amount of the material 60 for each coordinate, using a plurality of TC test material images 111 acquired in each count, for the each count of the TC test in which the cycle count (TC count) of the temperature load on the material 60 is N (N is an integer equal to or greater than 2). In other words, the hysteresis curve generation unit 121 generates N hysteresis curves corresponding to the TC count N for each coordinate of the material 60. The generated hysteresis curve may be stored in the storage unit 110.

Note that a coordinate of the material 60 is information indicating a position of each mesh when the material 60 is divided into meshes with predetermined granularity. The coordinate may be information indicating a point on the material 60, or information indicating an area having a predetermined size on the material 60. The area having a predetermined size on the material 60 may be a two-dimensional plane or a three-dimensional space.

The deformation amount one-dimensional information generation unit 122 performs processing of converting the hysteresis curve generated by the hysteresis curve generation unit 121 or the generated hysteresis curve stored in the storage unit 110 into one dimension. The deformation amount one-dimensional information generation unit 122 converts each of the N hysteresis curves corresponding to the TC count N obtained for each coordinate of the material 60 into one dimension to generate one-dimensional information (deformation amount one-dimensional information 151 to be described below) regarding the deformation amount of each coordinate of the material 60.

The deformation amount to TC count two-dimensional information generation unit 123 performs processing of arraying N pieces of one-dimensional information corresponding to the TC count N regarding the deformation amount of each coordinate of the material 60 with respect to the TC count N to convert the N pieces of one-dimensional information into two dimensions for each coordinate, the N pieces of one-dimensional information having been generated by the deformation amount one-dimensional information generation unit 122. The deformation amount to TC count two-dimensional information generation unit 123 generates two-dimensional information of the deformation amount of the material 60 with respect to the TC count, of each coordinate of the material 60, in other words, two-dimensional information of the deformation amount to TC count of the material 60 (deformation amount to TC count two-dimensional information 152 to be described below).

The TC count one-dimensional information generation unit 124 performs processing of converting the two-dimensional information of the deformation amount to TC count of each coordinate of the material 60 into one dimension, the two-dimensional information having been generated by the deformation amount to TC count two-dimensional information generation unit 123. The TC count one-dimensional information generation unit 124 converts each two-dimensional information of the deformation amount to TC count obtained for each coordinate of the material 60 into one dimension to generate one-dimensional information regarding the TC count (TC count one-dimensional information 153 to be described below) for each coordinate of the material 60. The one-dimensional information generated by the TC count one-dimensional information generation unit 124 can also be said to be information indicating ease of deformation at each coordinate of the material 60.

The TC count to coordinate two-dimensional information generation unit 125 performs processing of arraying the one-dimensional information regarding the TC count of each coordinate of the material 60 with respect to coordinates to convert pieces of the one-dimensional information into two dimensions, the pieces of one-dimensional information having been generated by the TC count one-dimensional information generation unit 124. The TC count to coordinate two-dimensional information generation unit 125 generates two-dimensional information of the TC count with respect to the coordinates of the material 60, in other words, two-dimensional information at the coordinates of the TC count to material 60 (TC count to coordinate two-dimensional information 154 to be described below).

The calculation unit 126 performs processing of analyzing the material 60 and processing of calculating physical property values of the material 60, using the two-dimensional information of the deformation amount to TC count at each coordinate of the material 60, which has been generated by the deformation amount to TC count two-dimensional information generation unit 123, for example. Here, the calculation unit 126 calculates the deformation amount of the material 60 using an FEM model 200 including the physical property values of the material 60 as parameters. The calculation unit 126 compares the calculated value of the deformation amount of the material 60 with the TC test material image 111, which is the measured value, and repeats the calculation while changing the combination of the physical property values until the calculated value matches the measured value or the difference from the measured value becomes equal to or less than a predetermined threshold value. The calculation unit 126 determines the physical property value with which the calculated value matches the measured value or the difference from the measured value becomes equal to or less than a predetermined threshold value as the physical property value of the material 60.

In this case, for example, the calculation unit 126 obtains the TC count at or near the elastic limit of the material 60 from the two-dimensional information of the deformation amount to TC count of the material 60. Then, the calculation unit 126 first performs the above-described repeated calculation while changing the combination of linear physical property values and determines the linear physical property values, and then performs the above-described repeated calculation while changing the combination of nonlinear physical property values, based on the obtained TC count and the two-dimensional information of the deformation amount to TC count.

Furthermore, the calculation unit 126 performs processing of calculating the physical property values of the material 60, using the two-dimensional information at the coordinates of the TC count to material 60, which has been generated by the TC count to coordinate two-dimensional information generation unit 125. Here, the calculation unit 126 calculates the deformation amount of the material 60 using the FEM model 200 including the physical property values of the material 60 as parameters. The calculation unit 126 compares the calculated value of the deformation amount of the material 60 with the TC test material image 111, which is the measured value, and repeats the calculation while changing the combination of the physical property values until the calculated value matches the measured value or the difference from the measured value becomes equal to or less than a predetermined threshold value. The calculation unit 126 determines the physical property value with which the calculated value matches the measured value or the difference from the measured value becomes equal to or less than a predetermined threshold value as the physical property value of the material 60.

In this case, for example, the calculation unit 126 divides the material 60 into predetermined areas, for example, areas with similar deformation behaviors, based on the two-dimensional information at the coordinates of the TC count to material 60, and further determines an order in which deformation of the divided areas occurs. Then, the calculation unit 126 performs the above-described repeated calculation while changing the combination of the physical property values for each of the divided areas in the determined order. For example, the calculation unit 126 performs the above-described repeated calculation while changing the combination of linear physical property values for the first area where deformation (mainly elastic deformation) occurs in an early period of the TC test (TC count). Then, the above-described repeated calculation is performed while changing the combination of nonlinear physical property values for the second and subsequent areas where deformation (mainly plastic deformation) occurs in middle and latter periods of the TC test (TC count).

Here, an example of the FEM model 200 used by the calculation unit 126 to calculate physical property values will be described.

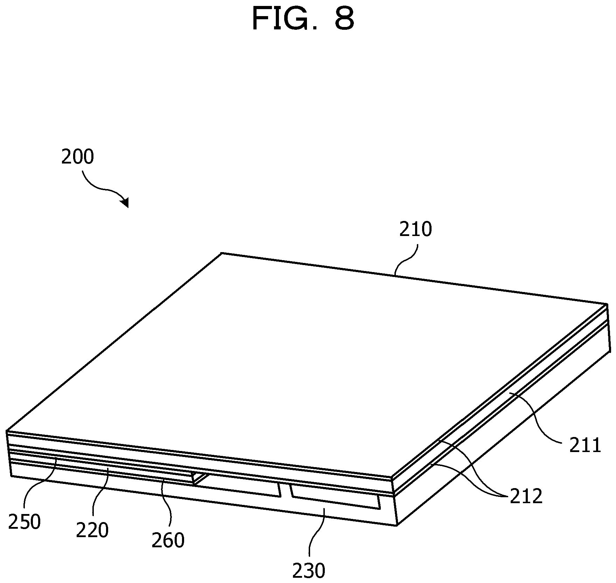

FIG. 8 is a view for describing an example of the FEM model according to the second embodiment.

For the material 60 to be evaluated by the material evaluation device 100, for example, an LSI package can be used. The FEM model 200 illustrated in FIG. 8 is obtained by modeling a structure of the LSI package of the material 60. For convenience, FIG. 8 illustrates a structure of one part (1/4 model) when the LSI package to be the material 60 is divided into four parts.

The FEM model 200 includes, as raw materials, a substrate 210 including a core 211 and a wiring layer 212, a semiconductor chip 220 mounted on the substrate 210, and a lid 230 that covers the substrate 210 and the semiconductor chip 220. The FEM model 200 further includes, as raw materials, a UF 250 provided between the substrate 210 and the semiconductor chip 220 and a TIM 260 provided between the semiconductor chip 220 and the lid 230. The FEM model 200 is created based on sizes and layouts of the raw materials obtained from CAD data or microscopic images.

The calculation unit 126 performs the processing of analyzing the material 60 and the processing of calculating the physical property values of the material 60, using such an FEM model 200.

Next, processing of the deformation amount one-dimensional information generation unit 122 will be described.

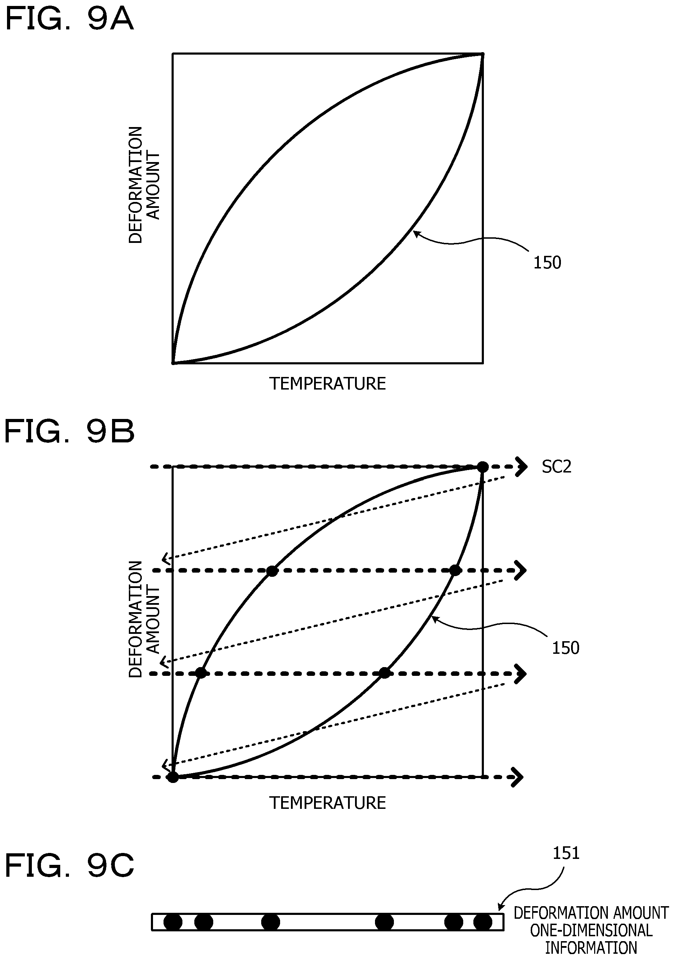

FIG. 9A, FIG. 9B, and FIG. 9C are diagrams for describing an example of processing of the deformation amount one-dimensional information generation unit according to the second embodiment. FIG. 9A is a diagram illustrating an example of a hysteresis curve, FIG. 9B is a diagram illustrating an example of scanning the hysteresis curve, and FIG. 9C is an example of deformation amount one-dimensional information obtained by scanning the hysteresis curve.

The deformation amount one-dimensional information generation unit 122 performs processing of converting the hysteresis curve 150 as illustrated in FIG. 9A into one dimension. The hysteresis curve 150 illustrated in FIG. 9A illustrates transition of the deformation amount at a certain coordinate of the material 60 at a certain count in the TC test performed with the TC count N for the material 60. The horizontal axis represents the temperature and the vertical axis represents the deformation amount. The hysteresis curve 150 is generated by the hysteresis curve generation unit 121.

The deformation amount one-dimensional information generation unit 122 scans the hysteresis curve 150 with a plurality of (four as an example) values of predetermined deformation amounts, as illustrated by thick dotted arrows SC2 in FIG. 9B, for example, to extract points on the hysteresis curve 150 when converting the hysteresis curve 150 into one dimension. A user can set predetermined deformation values for the values of the predetermined deformation amounts for scanning, in other words, scan resolution.

In scanning the hysteresis curve 150, points on the hysteresis curve are set to "1", and other points are set to "0". The deformation amount one-dimensional information generation unit 122 converts the hysteresis curve 150 into one dimension by superimposing information of "1" and "0" obtained by scanning the hysteresis curve 150 a plurality of times. In the example of FIG. 9B, the deformation amount one-dimensional information generation unit 122 extracts a total of 6 points as the information of "1" through the four times of scanning (the thick dotted arrows SC2), and generates deformation amount one-dimensional information 151 at a certain coordinate of the material 60, as illustrated in FIG. 9C.

The deformation amount one-dimensional information generation unit 122 performs similar processing for each of the N hysteresis curves 150 corresponding to the TC count N obtained for a certain coordinate of the material 60 to generate the deformation amount one-dimensional information 151 for each count of the TC count N. Furthermore, the deformation amount one-dimensional information generation unit 122 performs similar processing for each of other coordinates of the material 60 to generate the deformation amount one-dimensional information 151 for the each coordinate. Note that the scan resolution is assumed to be the same in the scanning for each hysteresis curve 150.

Next, processing of the deformation amount to TC count two-dimensional information generation unit 123 will be described.

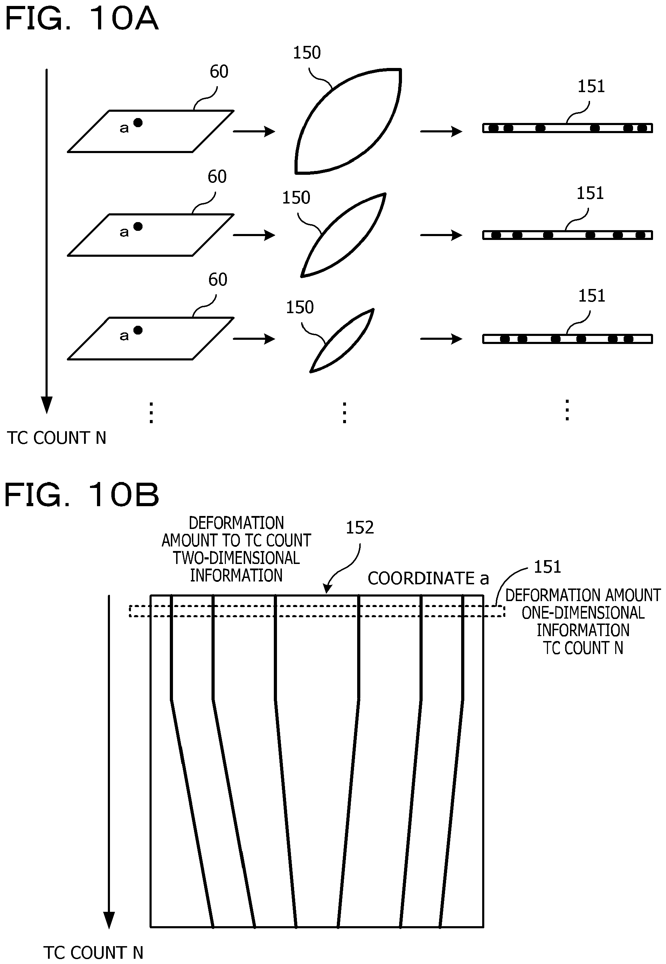

FIG. 10A and FIG. 10B are diagrams for describing an example of processing of the deformation amount to TC count two-dimensional information generation unit according to the second embodiment. FIG. 10A is a diagram illustrating an example of the deformation amount one-dimensional information generated for a certain coordinate of the material, and FIG. 10B is a diagram illustrating an example of the deformation amount to TC count two-dimensional information generated using the deformation amount one-dimensional information.

The deformation amount one-dimensional information generation unit 122 generates the deformation amount one-dimensional information 151 for each of the N hysteresis curves 150 corresponding to the TC count N obtained for the certain coordinate a of the material 60, as illustrated in FIG. 10A, for example. Note that, for the sake of convenience, FIG. 10A illustrates three hysteresis curves 150 and three pieces of the deformation amount one-dimensional information 151 corresponding to the TC count of three.

The deformation amount to TC count two-dimensional information generation unit 123 performs processing of arraying the deformation amount one-dimensional information 151 at the coordinate a of the material 60, of each count of the TC count N, with respect to the TC count N, to convert the pieces of deformation amount one-dimensional information 151 into two dimensions, the pieces of deformation amount one-dimensional information 151 having been generated by the deformation amount one-dimensional information generation unit 122. The deformation amount to TC count two-dimensional information generation unit 123 generates the deformation amount to TC count two-dimensional information 152 at the coordinate a of the material 60, as illustrated in FIG. 10B, by arranging the deformation amount one-dimensional information 151 at the coordinate a of the material 60 obtained at each count of the TC count N in the order of the TC count.

The deformation amount to TC count two-dimensional information generation unit 123 performs similar processing for each coordinate of the material 60 to generate the deformation amount to TC count two-dimensional information 152 for the each coordinate.

Here, the deformation amount to TC count two-dimensional information 152 will be further described.

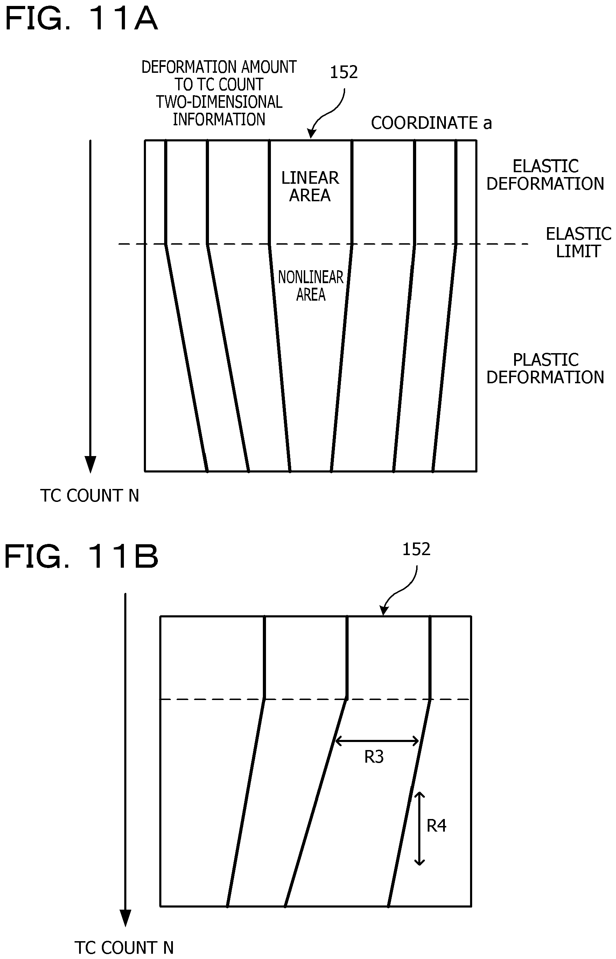

FIG. 11A and FIG. 11B are diagrams for describing an example of the deformation amount to TC count two-dimensional information according to the second embodiment. FIG. 11A is a diagram for describing a relationship between the deformation amount to TC count two-dimensional information and the material characteristics, and FIG. 11B is a diagram for describing a relationship between the deformation amount to TC count two-dimensional information and the material physical property values. Note that the deformation amount to TC count two-dimensional information in FIG. 11B is obtained by enlarging a right-side part of the deformation amount to TC count two-dimensional information in FIG. 11A.

FIG. 11A illustrates the deformation amount to TC count two-dimensional information 152 obtained by arranging the deformation amount one-dimensional information 151 at the certain coordinate a of the material 60 in the order of the TC count, as described above. In the deformation amount to TC count two-dimensional information 152, regarding the N hysteresis curves 150 corresponding to the TC count N at the coordinate a of the material 60, if the shapes of the hysteresis curves 150 at a certain count and at the next count of the certain count are the same as each other, transition of the deformation amount at the coordinate a of the material 60 is parallel to the axis direction of the TC count N. If the shapes of the hysteresis curves 150 at the certain count and the next count of the certain count are different from each other, the transition of the deformation amount at the coordinate a of the material 60 is not parallel to the axis direction of the TC count N, and becomes a direction inclined with respect to the axis direction of the TC count N.

In a case where the deformation at the coordinate a of the material 60 that occurs during the TC test is elastic deformation or is mainly elastic deformation, there is no difference in the shape of the hysteresis curve 150 depending on the TC count, or the difference is relatively small. Therefore, in the deformation amount to TC count two-dimensional information 152, the transition of the deformation amount at the coordinate a of the material 60 becomes parallel to the axis direction of the TC count N or becomes a relatively small inclination value with respect to the axis direction. Therefore, in an area where the transition of the deformation amount at the coordinate a of the material 60 becomes parallel to the axis direction of the TC count N or becomes a relatively small inclination value with respect to the axis direction in the deformation amount to TC count two-dimensional information 152, the deformation at the coordinate a of the material 60 during the TC test can be considered to be elastic deformation or mainly the elastic deformation. This area can be said to be a linear area in which the physical property values of the material 60 linearly changes by loading and unloading and the material 60 has linear characteristics.

Meanwhile, in a case where the deformation at the coordinate a of the material 60 that occurs during the TC test is plastic deformation or is mainly plastic deformation, the difference in the shape of the hysteresis curve 150 depending on the TC count is relatively large. Therefore, in the deformation amount to TC count two-dimensional information 152, the transition of the deformation amount at the coordinate a of the material 60 becomes a relatively large inclination value with respect to the axis direction of the TC count N. Therefore, in an area where the transition of the deformation amount at the coordinate a of the material 60 becomes a relatively large inclination value with respect to the axis direction of the TC count N in the deformation amount to TC count two-dimensional information 152, the deformation at the coordinate a of the material 60 during the TC test can be considered to be plastic deformation or mainly the plastic deformation. This area can be said to be a nonlinear area in which the physical property values of the material 60 nonlinearly changes by loading and unloading and the material 60 has nonlinear characteristics.

In the deformation amount to TC count two-dimensional information 152, the TC count at which the transition in which the deformation amount at the coordinate a of the material 60 becomes parallel to the axis direction of the TC count N or becomes a relatively small inclination value with respect to the axis direction changes to the transition with the relatively large inclination value with respect to the axis direction of the TC count N is a mutation point at which the tendency of transition changes. This mutation point can be considered to represent a boundary between the elastic deformation and the plastic deformation at the coordinate a of the material 60, that is, the TC count at or near a yield point indicating the elastic limit.

In this way, according to the deformation amount to TC count two-dimensional information 152, the area (linear area) of the TC count where the deformation at the coordinate a of the material 60 during the TC test is the elastic deformation or mainly the elastic deformation, and the area (nonlinear area) of the TC count where the deformation is the plastic deformation or mainly the plastic deformation can be divided.

The calculation unit 126 performs processing of calculating an inclination value with respect to the axis direction of the TC count N, of the transition of the deformation amount at the coordinate a of the material 60, using the deformation amount to TC count two-dimensional information 152, for example, and determining the TC count at which the calculated inclination value exceeds a predetermined threshold value as the mutation point (elastic limit or near the elastic limit).

Furthermore, at the TC count where the deformation is the plastic deformation or mainly the plastic deformation, destruction due to deformation tends to progress at the coordinate a of the material 60, and a hardening coefficient of a stress-strain curve can nonlinearly change. A change amount R3 in the cross direction in the deformation amount to TC count two-dimensional information 152, in other words, in the direction orthogonal to the axis direction of the TC count N, as illustrated in FIG. 11B, can be regarded as representing a strain accumulation amount, and the hardening coefficient of the coordinate a of the material 60 can be estimated from the strain accumulation amount. Furthermore, a change amount R4 in the longitudinal direction in the deformation amount to TC count two-dimensional information 152, in other words, in the axis direction of the TC count N, as illustrated in FIG. 11B, can be regarded as representing a strain accumulation speed, and an acceleration coefficient of the coordinate a of the material 60 can be estimated from the strain accumulation speed.

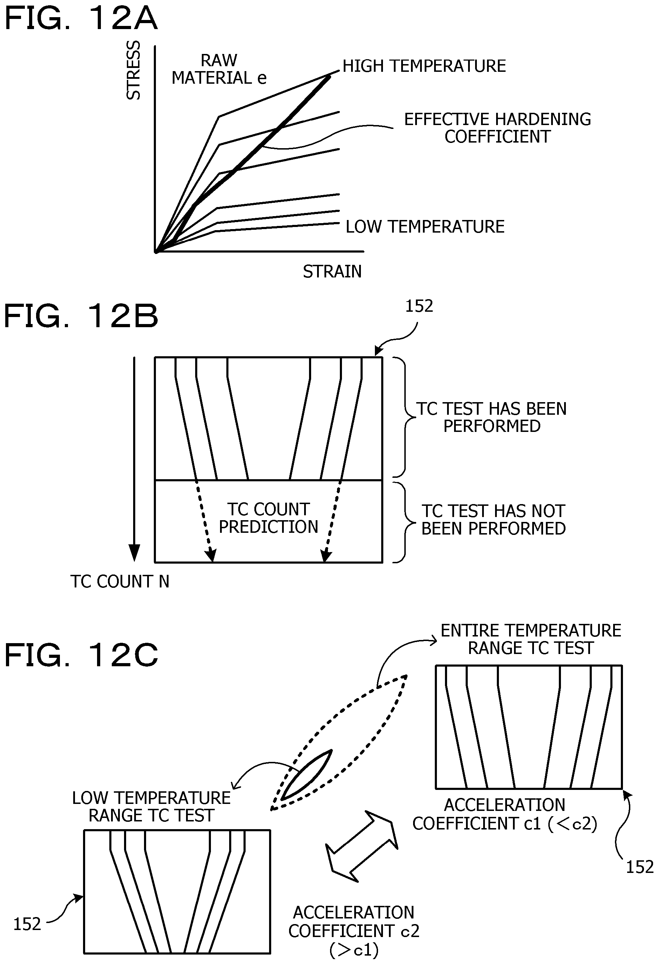

FIG. 12A, FIG. 12B, and FIG. 12C are diagrams for describing the hardening coefficient and the acceleration coefficient obtained from the deformation amount to TC count two-dimensional information according to the second embodiment. FIG. 12A is an explanatory diagram of the hardening coefficient, and FIG. 12B is an explanatory diagram of the acceleration coefficient.

As illustrated in FIG. 12A, the stress-strain curve varies depending on a raw material e of the material 60, and also varies depending on the temperature. Meanwhile, in the TC test, the temperature changes continuously. Therefore, it is not necessarily easy to prepare the stress-strain curve for each raw material e at each temperature in order to estimate the deformation and physical property values of the material 60. In contrast, in the deformation amount to TC count two-dimensional information 152, a strain accumulation amount at each TC count can be estimated from the transition of the deformation amount, and the hardening coefficient can be estimated from the strain accumulation amount. Therefore, an effective hardening coefficient in a temperature range of the TC test can be estimated without the stress-strain curve at each temperature for each raw material e.

Furthermore, a deformation rate of the material 60 per TC count in an area where the TC test has been performed can be obtained by using the acceleration coefficient obtained from the deformation amount to TC count two-dimensional information 152. From this deformation rate, as illustrated in FIG. 12B, how many more TC counts will be needed for the material 60 to reach a predetermined deformation amount, that is, the TC count for areas where the TC test has not been performed can be predicted.

Furthermore, as illustrated in FIG. 12C, an acceleration coefficient c1 obtained from the deformation amount to TC count two-dimensional information 152 of the TC test in the entire temperature range is smaller than an acceleration coefficient c2 obtained from the deformation amount to TC count two-dimensional information 152 of the TC test in a low temperature range. One deformation amount to TC count two-dimensional information 152 can be estimated from the other deformation amount to TC count two-dimensional information 152 of the TC tests in the entire temperature range and in the low temperature range based on such a relationship between the acceleration coefficient c1 and the acceleration coefficient c2. Therefore, for example, instead of the relatively time-consuming TC test in the entire temperature range, the TC test in the relatively short low temperature range is performed, and the deformation amount to TC count two-dimensional information 152 of the TC test in the entire temperature range can be estimated from the acceleration coefficient c2. By doing so, the time required for the TC test can be shortened.

Next, processing of the TC count one-dimensional information generation unit 124 will be described.

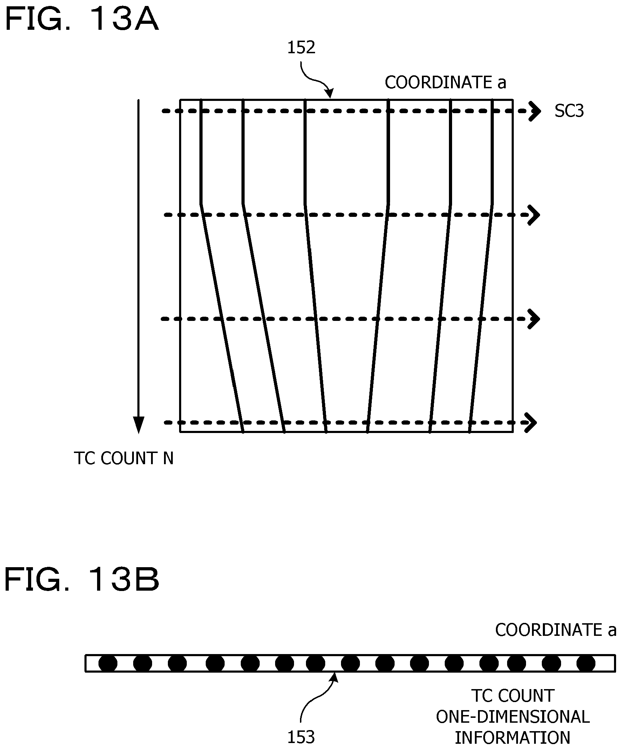

FIG. 13 A and FIG. 13B are diagrams for describing an example of processing of the TC count one-dimensional information generation unit according to the second embodiment. FIG. 13A is a diagram illustrating an example of scanning the deformation amount to TC count two-dimensional information, and FIG. 13B is a diagram illustrating an example of the TC count one-dimensional information obtained by scanning the deformation amount to TC count two-dimensional information.

The TC count one-dimensional information generation unit 124 performs processing of converting the deformation amount to TC count two-dimensional information 152 into one dimension. The TC count one-dimensional information generation unit 124 scans the deformation amount to TC count two-dimensional information 152 with a plurality of (four as an example) values of predetermined TC count, as illustrated by thick dotted arrows SC3 in FIG. 13A, for example, to extract points on the deformation amount to TC count two-dimensional information 152 when converting the deformation amount to TC count two-dimensional information 152 into one dimension. The user can set predetermined deformation values for the values of the predetermined TC count for scanning, in other words, scan resolution.

In scanning the deformation amount to TC count two-dimensional information 152, points on the deformation amount to TC count two-dimensional information 152 are set to "1", and other points are set to "0". The TC count one-dimensional information generation unit 124 converts the deformation amount to TC count two-dimensional information 152 into one dimension by superimposing information of "1" and "0" obtained by scanning the deformation amount to TC count two-dimensional information 152 a plurality of times. In the example of FIG. 13A, the TC count one-dimensional information generation unit 124 extracts a total of 24 points as the information of "1" through the four times of scanning (the thick dotted arrows SC3), and generates the TC count one-dimensional information 153 at the certain coordinate a of the material 60, as illustrated in FIG. 13B.

The TC count one-dimensional information generation unit 124 performs similar processing for each coordinate of the material 60 to generate the TC count one-dimensional information 153 for the each coordinate.

In the TC count one-dimensional information 153, the more the deformation amount to TC count two-dimensional information 152 transitions with a relatively large inclination value from a stage of a relatively early period of the TC count, the more the extraction points of the TC count are gathered in a center. In the TC count one-dimensional information 153, the more the deformation amount to TC count two-dimensional information 152 transitions with a relatively large inclination value from a stage of relatively middle and latter periods of the TC count, the more the extraction points of the TC count are gathered in an outer side.

Next, processing of the TC count to coordinate two-dimensional information generation unit 125 will be described.

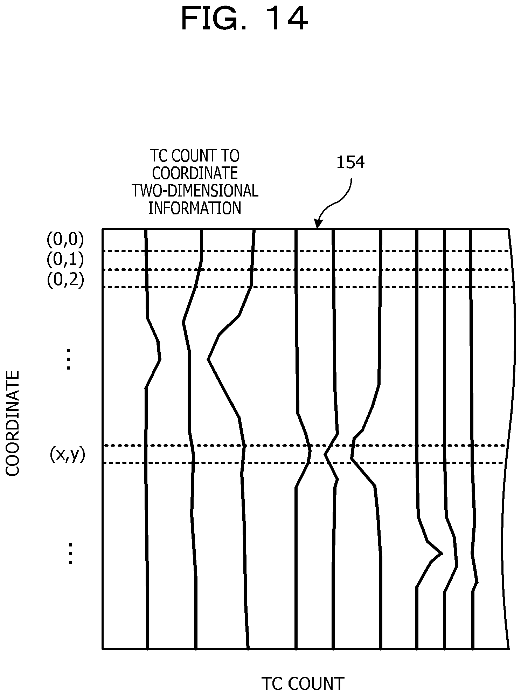

FIG. 14 is a diagram for describing an example of processing of the TC count to coordinate two-dimensional information generation unit according to the second embodiment. FIG. 14 is a diagram illustrating an example of the TC count to coordinate two-dimensional information generated using the TC count one-dimensional information.

The TC count to coordinate two-dimensional information generation unit 125 performs processing of arraying the TC count one-dimensional information 153 at each coordinate of the material 60 with respect to coordinates of the material 60 to convert pieces of the TC count one-dimensional information 153 into two dimensions, the pieces of TC count one-dimensional information 153 having been generated by the TC count one-dimensional information generation unit 124. The TC count to coordinate two-dimensional information generation unit 125 generates the TC count to coordinate two-dimensional information 154 of the material 60 as illustrated in FIG. 14 by arranging the TC count one-dimensional information 153 obtained for each coordinate of the material 60 by coordinates.

Next, processing of the calculation unit 126 will be described.

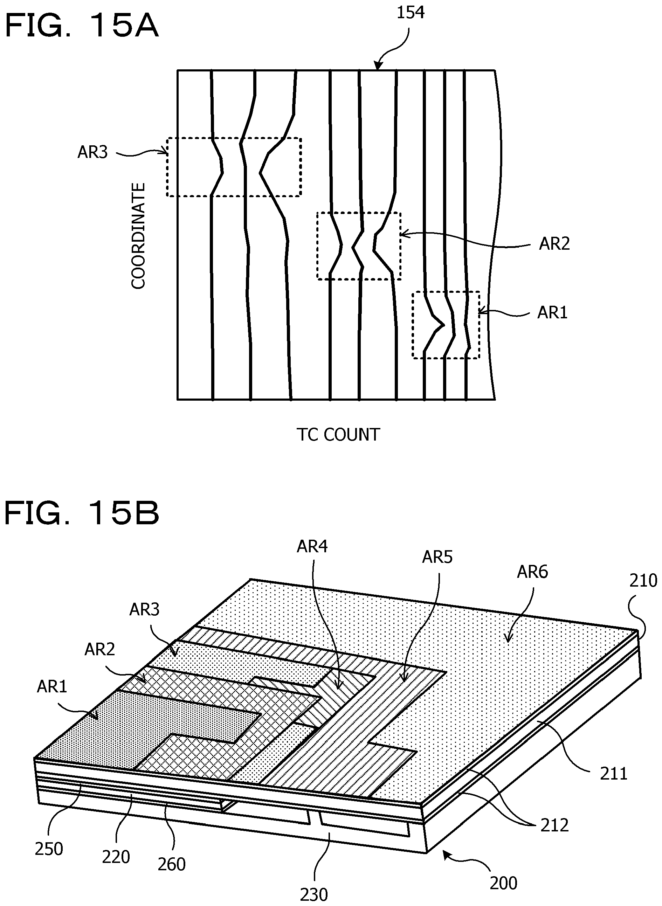

FIG. 15 A and FIG. 15B are diagrams for describing an example of processing of the calculation unit according to the second embodiment. FIG. 15A is a diagram illustrating an example of division of the TC count to coordinate two-dimensional information, and FIG. 15B is a diagram illustrating an example in which the division of the TC count to coordinate two-dimensional information is reflected in the FEM model. Note that FIG. 15A illustrates left-side part of the TC count to coordinate two-dimensional information 154.

The calculation unit 126 performs processing of dividing an area based on the transition for a TC count to two-dimensional information 154 generated by the TC count to coordinate two-dimensional information generation unit 125. For example, the calculation unit 126 divides the area based on a difference in sparseness and density and the inclination value of lines appearing in the TC count to coordinate two-dimensional information 154 illustrated in FIG. 15A. As an example, FIG. 15A illustrates three divided areas AR1, AR2, and AR3 (indicated by dotted frames). The division based on the difference in sparseness and density and the inclination value of the line in the TC count to coordinate two-dimensional information 154 can be performed using, for example, a machine learning method such as deep learning or clustering.

In the material 60, there may be no deformation at all in a certain coordinate group, while significant deformation may be seen at any of early period, middle period, or latter period of the TC test (TC count). In the TC test, the deformation of the material 60 locally occurs in an early period, undergoes plastic deformation, and leads to destruction. However, it is believed that the destruction does not occur all at once, but that small destructions lead to a large destruction. And once a destruction occurs, energy introduced with a load affects other coordinates to propagate the destruction. That is, a change in other coordinates becomes remarkable. In contrast, a change at the coordinates where the deformation has originally occurred is small. Considering this fact, it can be said that the behavior of deformation of the material 60 becomes similar for each area. From this point of view, regarding the material 60, the areas with similar deformation behavior, that is, set areas of coordinate groups in which the difference in sparseness and density and the inclination value of the line of the TC count to coordinate two-dimensional information 154 illustrated in FIG. 15A relatively substantially vary, for example, the area AR1, the area AR2, and the area AR3 can be divided. The calculation unit 126 extracts the set areas of the coordinate groups in which the difference in sparseness and density and the inclination value of the line of the TC count to coordinate two-dimensional information 154 relatively substantially vary, using a predetermined method, and divides the areas, in this manner.

Furthermore, the divided areas AR1, AR2, and AR3 of the TC count to coordinate two-dimensional information 154 illustrated in FIG. 15A can also be said to be temporally divided such as the early period, the middle period, and the latter period of the deformation of the material 60. This is because, in the TC count one-dimensional information 153 constituting the TC count to coordinate two-dimensional information 154, the more the deformation amount to TC count two-dimensional information 152 transitions with a relatively large inclination value from a stage of a relatively early period of the TC count, the more the extraction points of the TC count are gathered in a center. Furthermore, it is because, in the TC count one-dimensional information 153, the more the deformation amount to TC count two-dimensional information 152 transitions with a relatively large inclination value from a stage of relatively middle and latter periods of the TC count, the more the extraction points of the TC count are gathered in an outer side. For example, the area AR1 is divided into the early period of deformation of the material 60, the area AR2 is divided into the middle period of deformation of the material 60, and the area AR3 is divided into the latter period of deformation of the material 60. The calculation unit 126 further temporally divides the areas that have been divided by extracting the set of the coordinate groups in which the difference in sparseness and density and the inclination value of the line of the TC count to coordinate two-dimensional information 154 relatively substantially vary, and determines the order of divided areas.

The calculation unit 126 reflects information of the areas divided from the TC count to coordinate two-dimensional information 154 in the FEM model 200 of the material 60 as illustrated in FIG. 15B. FIG. 15B illustrates an example in which the material 60 is divided into six areas AR1 to AR6 and the six divided areas AR1 to AR6 are reflected in the FEM model 200. The calculation unit 126 calculates the physical property values for each of the reflected areas AR1 to AR6 and in order from the determined earliest order, using the FEM model 200.

The material 60 tends to elastically deform at the TC count in a relatively early period before the elastic limit and plastically deform at the TC count in the middle or latter period after the elastic limit. In calculating the physical property values using the FEM model 200, the calculation unit 126 first calculates the linear physical property values of each raw material of the material 60, for example, the linear expansion coefficient, Young's modulus, and Poisson's ratio, for the first area in order, which is believed to have the elastic deformation or mainly the elastic deformation. Here, the calculation unit 126 calculates the deformation amount of the material 60 using the FEM model 200. The calculation unit 126 compares the calculated value of the deformation amount of the material 60 with the TC test material image 111, which is the measured value, and repeats the calculation while changing the combination of the linear physical property values until the calculated value matches the measured value or the difference from the measured value becomes equal to or less than a predetermined threshold value. The calculation unit 126 determines the physical property value with which the calculated value matches the measured value or the difference from the measured value becomes equal to or less than the predetermined threshold value as the linear physical property value of each raw material of the material 60.

After the determination of the linear physical property value, the calculation unit 126 calculates the nonlinear physical property values of each raw material of the material 60, for example, a hardening coefficient, for the second area in order, which is believed to have the plastic deformation or mainly the plastic deformation. Here, the calculation unit 126 calculates the deformation amount of the material 60 using the FEM model 200. The calculation unit 126 compares the calculated value of the deformation amount of the material 60 with the TC test material image 111, which is the measured value, and repeats the calculation while changing the combination of the nonlinear physical property values until the calculated value matches the measured value or the difference from the measured value becomes equal to or less than a predetermined threshold value. The calculation unit 126 determines the physical property value with which the calculated value matches the measured value or the difference from the measured value becomes equal to or less than the predetermined threshold value as the nonlinear physical property value of each raw material of the second area of the material 60. The calculation unit 126 also performs similar processing for the third and subsequent areas in the order, and determines the nonlinear physical property value of each raw material in each area.

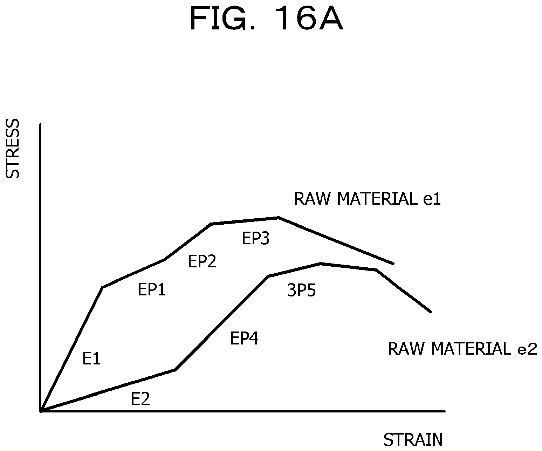

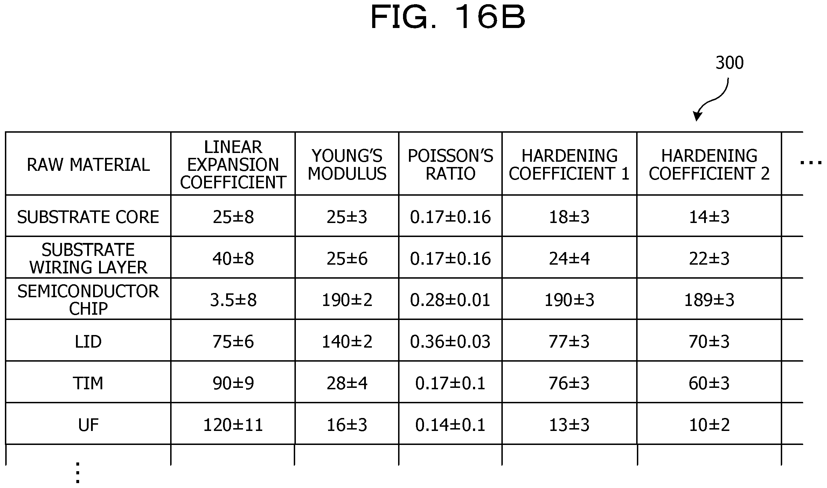

FIG. 16A and FIG. 16B are diagrams illustrating a calculation example of physical property values according to the second embodiment. FIG. 16A is a diagram illustrating an example of a relationship between stress and strain for each raw material contained in the material, and FIG. 16B is a diagram illustrating an example of physical property values for each raw material contained in the material.

By the above-described processing, the calculation unit 126 calculates the linear physical property values of the raw material contained in the material 60 for the first area having the elastic deformation or mainly the elastic deformation among the divided areas of the material 60. For example, a linear physical property value E1 of a raw material e1 and a linear physical property value E2 of a raw material e2 as illustrated in FIG. 16A are calculated.

Furthermore, the calculation unit 126 calculates the nonlinear physical property values of the raw material contained in the material 60 for the second and subsequent areas having the plastic deformation or mainly the plastic deformation among the divided areas of the material 60. For example, hardening coefficients EP1, EP2, EP3, and the like of the stress-strain curve of the raw material e1 and hardening coefficients EP4, EP5, and the like of the stress-strain curve of the raw material e2 as illustrated in FIG. 16A are calculated. Even if there is no stress-strain curve (FIG. 12A) for each temperature of each of the raw materials e1 and e2, the hardening coefficients and nonlinear physical property values can be calculated.

By the above-described processing, the physical property values of each raw material of the material 60 are calculated as listed in a physical property value table 300 illustrated in FIG. 16B. For example, in a case where the material 60 is an LSI package, the physical property values are calculated for each raw material such as the core 211 and the wiring layer 212 of the substrate 210, the semiconductor chip 220, the lid 230, the TIM 260, and the UF 250 illustrated in the FEM model 200 in FIG. 8. In the example of FIG. 16B, the linear physical property values are the linear expansion coefficient, Young's modulus, and Poisson's ratio, and the nonlinear physical property values are the hardening coefficient 1 and hardening coefficient 2. The content of the physical property value table 300 may be stored in the storage unit 110. Note that the types of raw materials and physical property values are not limited to those illustrated in FIG. 16B.

As described above, according to the material evaluation device 100, the material 60 is divided spatially and temporally into areas where the deformation behavior during the TC test is similar, so that the granularity in calculating the physical property values using the FEM model 200 becomes able to be adjusted. Therefore, a calculation amount for calculating the physical property values of the material 60 can be significantly reduced. According to the material evaluation device 100, the physical property values required for constructing the model that reproduces the deformation of the material 60 can be accurately and efficiently acquired, and the model can be accurately and efficiently constructed.

For example, if the measured values and the calculated values of the FEM model 200 were compared for all of 256 pixels by 256 pixels (65536 pixels) of the TC test material image 111 of the material 60, 65536 dimensions of calculation would be required. In the case of the technology using an HOG feature as described in Document 1 above, 100 dimensions of calculation are required. In contrast, in the case where the FEM model 200 is divided into six areas by the material evaluation device 100, six dimensions of calculation are sufficient. As a result, according to the material evaluation device 100, the calculation amount can be reduced by 99.99% compared to all-pixel comparison, and by 94% compared to the technology using an HOG feature.

Note that the calculation unit 126 may calculate the physical property values of the material 60 based on the deformation amount to TC count two-dimensional information 152 illustrated in FIGs. 10 and 11, which has been generated by the deformation amount to TC count two-dimensional information generation unit 123. For example, the calculation unit 126 uses the TC test material image 111 of the TC count in the linear area having mainly the elastic deformation for each coordinate and each raw material of the material 60 as the measured value, and repeats the calculation while changing the combination of the linear physical property values until the difference between the calculated value and the measured value of the FEM model 200 becomes equal to or less than a predetermined threshold value. Therefore, the linear physical property values of the material 60 are determined. Thereafter, the calculation unit 126 uses the TC test material image 111 of the TC count in the nonlinear area having mainly the plastic deformation for each coordinate and each raw material of the material 60 as the measured value, and repeats the calculation while changing the combination of the nonlinear physical property values until the difference between the calculated value and the measured value of the FEM model 200 becomes equal to or less than a predetermined threshold value. Therefore, the nonlinear physical property values of the material 60 are determined. The calculation unit 126 may thus calculate the linear physical property values and the nonlinear physical property values of the material 60 based on the deformation amount to TC count two-dimensional information 152.

Next, processing of the material evaluation device 100 will be described.

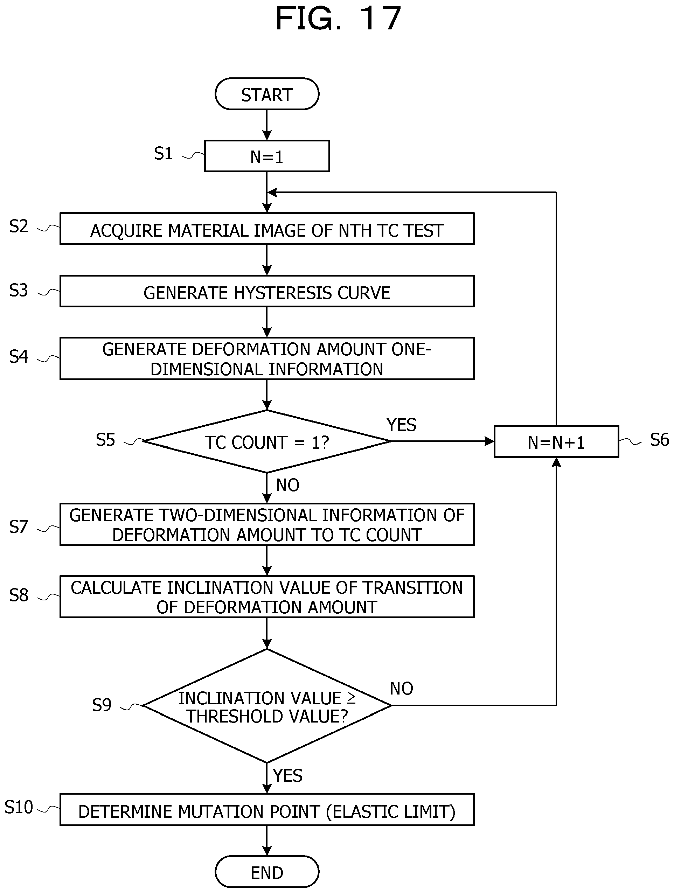

FIG. 17 is a flowchart illustrating an example of a mutation point determination processing flow according to the second embodiment.

FIG. 17 illustrates an example of a processing flow for determining, in real time, the mutation point, which is the elastic limit at which the material 60 changes from elastic deformation to plastic deformation, while performing the TC test. The material evaluation device 100 executes processing described in steps S1 to S10.

(S1) The material evaluation device 100 performs the first TC test (TC count N = 1). The material evaluation device 100 stores, in the storage unit 110, a plurality of material images (TC test material images) 111 acquired in the TC test at the TC count N = 1.

(S2) The material evaluation device 100 refers to the storage unit 110 and acquires the TC test material image 111 at the TC count N = 1 by the hysteresis curve generation unit 121.

(S3) The material evaluation device 100 generates, by the hysteresis curve generation unit 121, the hysteresis curve 150 indicating the transition of the deformation amount at the coordinate a of the material 60 using the TC test material image 111 at TC count N = 1. The hysteresis curve 150 has the temperature on the horizontal axis and the deformation amount on the vertical axis.

(S4) The material evaluation device 100 scans, with a value of a predetermined deformation amount, and converts, by the deformation amount one-dimensional information generation unit 122, the generated hysteresis curve 150 into one dimension to generate the deformation amount one-dimensional information 151.

(S5) The material evaluation device 100 determines whether the TC test is the first time (TC count = 1) or not.

(S6) In a case of determining that the TC test is the first time, the material evaluation device 100 adds 1 to the TC count N and performs the second TC test (TC count N = 2), and stores the acquired TC test material image 111 in the storage unit 110. The material evaluation device 100 executes the processing of steps S2 to S4 using the TC test material image 111 at the TC count N = 2.

(S7) In a case of determining that the TC test is not the first time in step S5, the material evaluation device 100 arrays, by the deformation amount to TC count two-dimensional information generation unit 123, the deformation amount one-dimensional information 151 at the coordinate a of the material 60 with respect to the TC count N and converts the deformation amount one-dimensional information 151 into two dimensions to generate the deformation amount to TC count two-dimensional information 152.

(S8) The material evaluation device 100 calculates, by the calculation unit 126, the inclination value of the transition of the deformation amount at the coordinate a of the material 60 in the generated deformation amount to TC count two-dimensional information 152.

(S9) The material evaluation device 100 determines, by the calculation unit 126, whether the calculated inclination value is equal to or larger than the predetermined threshold value or not. In a case of determining that the calculated inclination value becomes less than the threshold value, the material evaluation device 100 returns to step S6, adds 1 to the TC count N and performs the TC test, and executes the processing of steps S2 to S8 using the acquired TC test material image 111.

(S10) In a case of determining that the calculated inclination value becomes equal to or larger than the threshold value in step S9, the material evaluation device 100 determines, by the calculation unit 126, the TC count N at which the inclination value has become equal to or larger than the threshold value as the mutation point, that is, the elastic limit.

According to the mutation point determination processing flow illustrated in FIG. 17, the mutation point can be detected in real time during the TC test. Therefore, it becomes possible to reduce the number of TC tests, shorten the time, and efficiently calculate the physical property values of the material 60, and efficiently construct the model that reproduces the material 60.

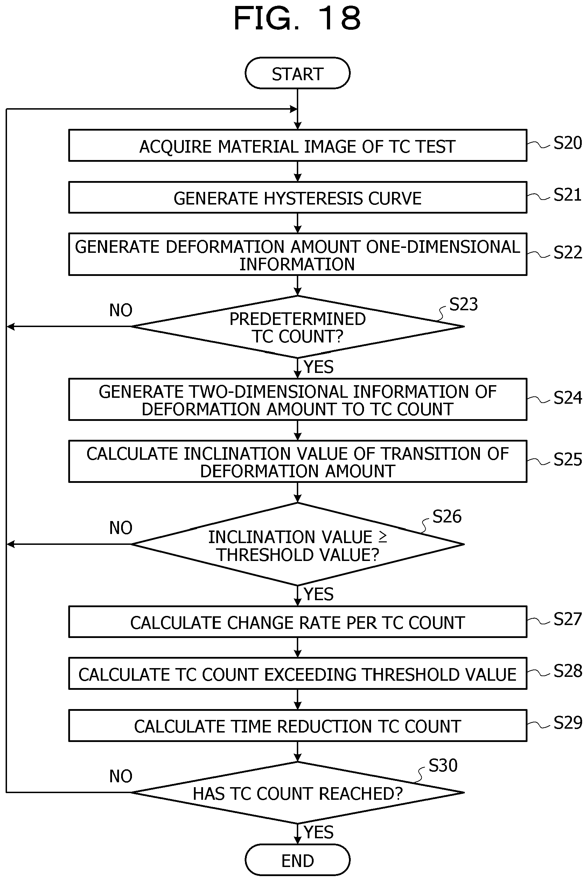

FIG. 18 is a flowchart illustrating an example of a TC count prediction processing flow according to the second embodiment.

FIG. 18 illustrates an example of a processing flow of reducing time and performing the TC test while calculating the number of the TC tests required to be further performed until the material 60 reaches a predetermined deformation amount from the information regarding the deformation of the material 60 for which the TC test has been performed. The material evaluation device 100 executes processing described in steps S20 to S30.

(S20) The material evaluation device 100 acquires, by the hysteresis curve generation unit 121, a plurality of material images (TC test material images) 111 acquired in the TC test by referring to the storage unit 110.

(S21) The material evaluation device 100 generates, by the hysteresis curve generation unit 121, the hysteresis curve 150 indicating the transition of the deformation amount at the coordinate a of the material 60 using the TC test material images 111. The hysteresis curve 150 has the temperature on the horizontal axis and the deformation amount on the vertical axis.

(S22) The material evaluation device 100 scans, with a value of a predetermined deformation amount, and converts, by the deformation amount one-dimensional information generation unit 122, the generated hysteresis curve 150 into one dimension to generate deformation amount one-dimensional information 151.

(S23) The material evaluation device 100 determines whether the TC count N is a predetermined number of times or not. The material evaluation device 100 executes the processing of steps S20 to S22 until the TC count N reaches the predetermined number of times.

(S24) In a case of determining that the TC count N is a predetermined number of times in step S23, the material evaluation device 100 arrays, by the deformation amount to TC count two-dimensional information generation unit 123, the deformation amount one-dimensional information 151 at the coordinate a of the material 60 with respect to the TC count N and converts the deformation amount one-dimensional information 151 into two dimensions to generate the deformation amount to TC count two-dimensional information 152.

(S25) The material evaluation device 100 calculates, by the calculation unit 126, the inclination value of the transition of the deformation amount at the coordinate a of the material 60 in the generated deformation amount to TC count two-dimensional information 152.

(S26) The material evaluation device 100 determines, by the calculation unit 126, whether the calculated inclination value is equal to or larger than the predetermined threshold value or not. The material evaluation device 100 executes the processing of steps S20 to S25 until the calculated inclination value becomes equal to or larger than the threshold value.

(S27) In a case of determining that the calculated inclination value becomes equal to or larger than the threshold value in step S26, the material evaluation device 100 calculates, by the calculation unit 126, a change rate (acceleration coefficient) per TC count.

(S28) The material evaluation device 100 calculates, by the calculation unit 126, the TC count required to reach the threshold value by dividing the threshold value of a predetermined deformation amount determined by the user by the change rate per TC count calculated in step S27. For example, the threshold value of the predetermined deformation amount is assumed to be 0.2%.

(S29) The material evaluation device 100 calculates, by the calculation unit 126, a time reduction TC count by multiplying the TC count calculated in step 28 by a predetermined time reduction rate determined by the user. For example, the predetermined time reduction rate is assumed to be 50%.

(S30) The material evaluation device 100 determines whether the TC count N has reached the time reduction TC count calculated in step S29 or not, and executes the processing of steps 20 to S29 until the TC count N reaches the time reduction TC count.

According to the TC count prediction processing flow illustrated in FIG. 18, it is possible to reduce the number of TC tests, shorten the time, and efficiently calculate the physical property values of the material 60. Therefore, it becomes possible to efficiently construct the model that reproduces the material 60.

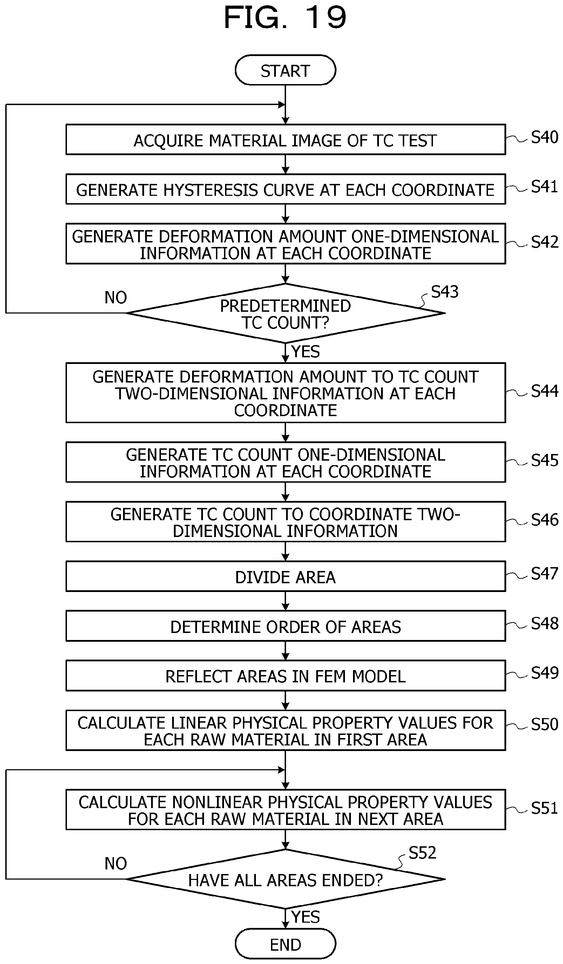

FIG. 19 is a flowchart illustrating an example of a physical property value calculation processing flow according to the second embodiment.

FIG. 19 illustrates an example of a processing flow of spatially and temporally dividing the area of the material 60, adjusting granularity, and calculating the linear and nonlinear physical property values of the material 60. The material evaluation device 100 executes processing described in steps S40 to S52.

(S40) The material evaluation device 100 acquires, by the hysteresis curve generation unit 121, a plurality of material images (TC test material images) 111 acquired in the TC test by referring to the storage unit 110.

(S41) The material evaluation device 100 generates, by the hysteresis curve generation unit 121, the hysteresis curve 150 indicating the transition of the deformation amount at each coordinate of the material 60 using the TC test material images 111. Each hysteresis curve 150 has the temperature on the horizontal axis and the deformation amount on the vertical axis.

(S42) The material evaluation device 100 scans, with a value of a predetermined deformation amount, and converts, by the deformation amount one-dimensional information generation unit 122, each of the generated hysteresis curves 150 into one dimension to generate deformation amount one-dimensional information 151 at each coordinate.

(S43) The material evaluation device 100 determines whether the TC count N is a predetermined number of times or not. The material evaluation device 100 executes the processing of steps S40 to S42 until the TC count N reaches the predetermined number of times.

(S44) In a case of determining that the TC count N is a predetermined number of times in step S43, the material evaluation device 100 arrays, by the deformation amount to TC count two-dimensional information generation unit 123, the deformation amount one-dimensional information 151 at each coordinate of the material 60 with respect to the TC count N and converts the deformation amount one-dimensional information 151 into two dimensions to generate the deformation amount to TC count two-dimensional information 152 at each coordinate.

(S45) The material evaluation device 100 scans, with a value of a predetermined TC count, and converts, by the TC count one-dimensional information generation unit 124, the generated deformation amount to TC count two-dimensional information 152 at each coordinate of the material 60 into one dimension to generate the TC count one-dimensional information 153 at each coordinate.

(S46) The material evaluation device 100 arrays, by the TC count to coordinate two-dimensional information generation unit 125, the generated TC count one-dimensional information 153 at each coordinate of the material 60 with respect to coordinates and converts the TC count one-dimensional information 153 into two dimensions to generate the TC count to coordinate two-dimensional information 154.

(S47) The material evaluation device 100 divides, by the calculation unit 126, the areas in which the deformation behavior is similar in the material 60 based on the difference in sparseness and density and the inclination value of the lines appearing in the generated TC count to coordinate two-dimensional information 154.

(S48) The material evaluation device 100 determines, by the calculation unit 126, the order of the areas of the material 60 divided in step S47.

(S49) The material evaluation device 100 reflects, by the calculation unit 126, the areas of the material 60, divided in step S47 and the order of which has been determined in step S48, in the FEM model 200.

(S50) The material evaluation device 100 calculates, by the calculation unit 126, the linear physical property values for each raw material contained in the material 60, for the first divided area, using the FEM model 200. The calculation unit 126 compares the calculated value of the FEM model 200 with the measured value of the TC test material image 111, repeats the calculation while changing the combination of the physical property values until the calculated value matches the measured value or the difference from the measured value becomes equal or less than a predetermined threshold value, and calculates the linear physical property values.

(S51) The material evaluation device 100 calculates, by the calculation unit 126, the nonlinear physical property values for each raw material contained in the material 60, for the next divided area, using the FEM model 200. The calculation unit 126 compares the calculated value of the FEM model 200 with the measured value of the TC test material image 111, repeats the calculation while changing the combination of the physical property values until the calculated value matches the measured value or the difference from the measured value becomes equal or less than a predetermined threshold value, and calculates the nonlinear physical property values.

(S52) The material evaluation device 100 executes the processing of step S51 for all the divided areas of the material 60.

According to the physical property value calculation processing flow illustrated in FIG. 19, it is possible to divide the material 60 and adjust the granularity, reduce the calculation amount required for calculating the physical property values, and efficiently calculate the physical property values. Therefore, it becomes possible to efficiently construct the model that reproduces the material 60.

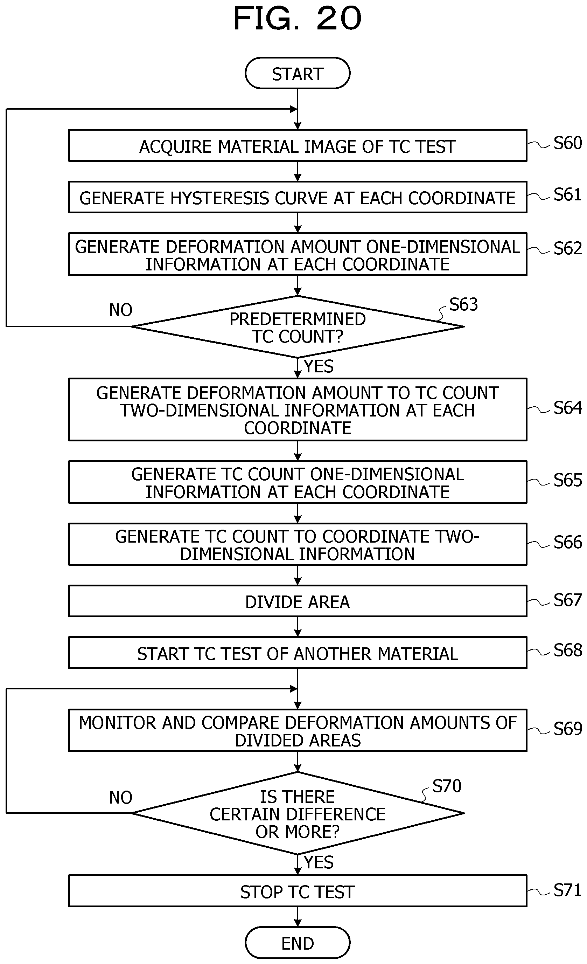

FIG. 20 is a flowchart illustrating an example of a TC test monitoring processing flow according to the second embodiment.

FIG. 20 illustrates an example of a processing flow of monitoring and comparing TC tests of different materials from the information of deformation of the material 60 and determining whether to continue or stop the TC tests of the different materials. The material evaluation device 100 executes processing described in steps S60 to S71.

(S60) The material evaluation device 100 acquires, by the hysteresis curve generation unit 121, a plurality of material images (TC test material images) 111 acquired in the TC test by referring to the storage unit 110.

(S61) The material evaluation device 100 generates, by the hysteresis curve generation unit 121, the hysteresis curve 150 indicating the transition of the deformation amount at each coordinate of the material 60 using the TC test material images 111. Each hysteresis curve 150 has the temperature on the horizontal axis and the deformation amount on the vertical axis.

(S62) The material evaluation device 100 scans, with a value of a predetermined deformation amount, and converts, by the deformation amount one-dimensional information generation unit 122, each of the generated hysteresis curves 150 into one dimension to generate deformation amount one-dimensional information 151 at each coordinate.

(S63) The material evaluation device 100 determines whether the TC count N is a predetermined number of times or not. The material evaluation device 100 executes the processing of steps S60 to S62 until the TC count N reaches the predetermined number of times.

(S64) In a case of determining that the TC count N is a predetermined number of times in step S43, the material evaluation device 100 arrays, by the deformation amount to TC count two-dimensional information generation unit 123, the deformation amount one-dimensional information 151 at each coordinate of the material 60 with respect to the TC count N and converts the deformation amount one-dimensional information 151 into two dimensions to generate the deformation amount to TC count two-dimensional information 152 at each coordinate.

(S65) The material evaluation device 100 scans, with a value of a predetermined TC count, and converts, by the TC count one-dimensional information generation unit 124, the generated deformation amount to TC count two-dimensional information 152 at each coordinate of the material 60 into one dimension to generate TC count one-dimensional information 153 at each coordinate.

(S66) The material evaluation device 100 arrays, by the TC count to coordinate two-dimensional information generation unit 125, the generated TC count one-dimensional information 153 at each coordinate of the material 60 with respect to coordinates and converts the TC count one-dimensional information 153 into two dimensions to generate the TC count to coordinate two-dimensional information 154.

(S67) The material evaluation device 100 divides, by the calculation unit 126, the areas in which the deformation behavior is similar in the material 60 based on the difference in sparseness and density and the inclination value of the lines appearing in the generated TC count to coordinate two-dimensional information 154.

(S68) The material evaluation device 100 starts the TC test of another material different from the material 60.

(S69) The material evaluation device 100 monitors the deformation amount of an area of the another material corresponding to the area divided in the material 60, and compares the deformation amount with the deformation amount obtained in the material 60.

(S70) The material evaluation device 100 determines whether there is a certain difference or more between the deformation amount of the material 60 and the deformation amount of the another material or not. In a case of determining that there is no certain difference or more in the deformation amount between the material 60 and the another material, the material evaluation device 100 continues the TC test of the another material, returns to step S69, and executes the processing.

(S71) In a case of determining that there is a certain difference or more in the deformation amount between the material 60 and the another material, the material evaluation device 100 stops the TC test of the another material.

According to the TC test monitoring processing flow illustrated in FIG. 20, it becomes possible to capture in advance, deformation of another material, for example, significant deformation or destruction based on the TC test record of the material 60, predicts the number and time of the TC tests, and suppress an excessive number of TC tests. Therefore, it becomes possible to efficiently calculate the physical property values of the material 60, and efficiently construct the model that reproduces the material 60.

Note that the processing functions that the material evaluation device 100 has as described above can be implemented by a computer. In that case, a program describing the processing content of the functions that the material evaluation device 100 is supposed to have is provided, and the above processing functions are implemented on the computer by the execution of the program on the computer. The program describing the processing content may be recorded on a computer-readable recording medium. The computer-readable recording medium includes a magnetic storage device, an optical disk, a magneto-optical recording medium, a semiconductor memory, or the like. The magnetic storage device includes an HDD, a flexible disk, a magnetic tape, or the like. The optical disk includes a CD, a DVD, or the like. The magneto-optical recording media includes an MO or the like.

In a case where the program is distributed, for example, a portable recording medium such as a DVD, a CD-ROM, and the like in which the program is recorded is sold. Furthermore, it is also possible to store the program in a storage device of a server computer and transfer the program from the server computer to another computer via a network.

The computer that executes the program stores, for example, the program recorded on the portable recording medium or the program transferred from the server computer in its own storage device. Then, the computer reads the program from the storage device of the computer and executes processing according to the program. Note that the computer may also read the program directly from the portable recording medium and execute processing according to the program. The computer can also execute processing according to the received program every time the program is transferred from the server computer connected via a network.

The above description has exemplified the method of analyzing the material and calculating the physical property values based on the hysteresis curve representing the deformation behavior of the material during the TC test in which one cycle of the temperature load is repeatedly given to the material. In addition, the above method can be applied to the analysis of materials, the calculation of physical property values, and the modeling of materials in which the hysteresis curve for another physical quantity is obtained by repeatedly applying a certain physical quantity. For example, the above method can be applied to the modeling of magnetic characteristics of a magnetic material, that is, the analysis of the magnetic material, the calculation of the physical property values, and the modeling from which a hysteresis curve indicating the relationship between a magnetic field and magnetic flux density can be obtained. Alternatively, the above method can be applied to modeling of charge/discharge characteristics of battery materials, in other words, analysis of battery materials, calculation of physical property values, and modeling from which a hysteresis curve indicating the relationship between a state of charge and a voltage or the like can be obtained.

The above description merely provides examples. Moreover, many modifications and variations can be made by those skilled in the art, and the present invention is not limited to the precise arrangements and applications illustrated and described above. All corresponding variations and equivalents are considered to be within the scope of the invention according to the appended claims and equivalents thereof.

### REFERENCE SIGNS LIST

1, 100Material evaluation device
10, 110Storage unit
20, 120Processing unit
30, 60Material
40, 150, 704, 705, 706Hysteresis curve
41One-dimensional information
42Two-dimensional information
50Material evaluation system
70Measurement device
71Stage
72Camera
80Temperature control device
90Imaging control device
101Processor
102RAM
103HDD
104Image signal processing unit
104aDisplay
105Input signal processing unit
105aInput device
106Reading device
106aRecording medium
107Communication interface
111TC test material image
121Hysteresis curve generation unit
122Deformation amount one-dimensional information generation unit
123Deformation amount to TC count two-dimensional information generation unit
124TC count one-dimensional information generation unit
125TC count to coordinate two-dimensional information generation unit
126Calculation unit
130Input device
140Display device
151Deformation amount one-dimensional information
152Deformation amount to TC count two-dimensional information
153TC count one-dimensional information
154TC count to coordinate two-dimensional information
200FEM model
210, 510Substrate
211Core
212Wiring layer
220, 520Semiconductor chip
230, 530Lid
250, 550UF
260, 560TIM
300Physical property value table
400Temperature cycle
500LSI package
520aPosition
540Bump
570Adhesive
601, 602, 603, 604, 605, 606Material image

## Claims

1. A material evaluation device comprising:
a storage unit configured to store N (N is an integer equal to or greater than 2) hysteresis curves that each represent a change in a second physical quantity of each time with respect to a change in a first physical quantity of N times at at least one of a plurality of positions of a material; and
a processing unit configured to refer to the storage unit, scan each of the N hysteresis curves with a value of the predetermined second physical quantity, for at least one of the plurality of positions, to extract a point, and one-dimensionally array the extracted points to generate one-dimensional information regarding the second physical quantity, and
calculate a physical property value of the material using the generated one-dimensional information regarding the second physical quantity.

2. The material evaluation device according to claim 1, wherein
the processing unit
calculates a change amount between pieces of one-dimensional information regarding the second physical quantity, the one-dimensional information having been generated for each of the hysteresis curve of the second physical quantity with respect to an (N - 1)th change in the first physical quantity, and the hysteresis curve of the second physical quantity with respect to an Nth change in the first physical quantity,
determines that the material has a linear characteristic by the (N - 1)th and Nth changes in the first physical quantity in a case where the calculated change amount is less than a first threshold value,
determines that the material has a linear characteristic by the (N - 1)th change in the first physical quantity, and the material has a nonlinear characteristic by the Nth change in the first physical quantity, in a case where the calculated change amount is equal to or larger than the first threshold value, and
calculates the physical property value of the material based on a determined result.

3. The material evaluation device according to claim 1, wherein
the processing unit
calculates a change amount between pieces of one-dimensional information regarding the second physical quantity, the one-dimensional information having been generated for each of the N hysteresis curves for at least one of the plurality of positions, and
calculates a predicted value of a count of (N + 1)th or subsequent changes in the first physical quantity until the second physical quantity reaches a second threshold value, using the calculated change amount.

4. The material evaluation device according to claim 1, wherein
the storage unit stores the N hysteresis curves at each of the plurality of positions of the material; and
the processing unit
generates two-dimensional information of the second physical quantity to the count, in which one-dimensional information regarding the second physical quantity generated for each of the N hysteresis curves is arrayed with respect to the count of changes in the first physical quantity, for each of the plurality of positions,
scans the two-dimensional information of the second physical quantity to the count generated for each of the plurality of positions with a value of the predetermined count to extract a point, and one-dimensionally arrays the extracted points to generate one-dimensional information regarding the count,
generates two-dimensional information of the count to the plurality of positions, in which the one-dimensional information regarding the count generated for each of the plurality of positions is arrayed with respect to the plurality of positions, and
calculates the physical property value of the material, using the generated two-dimensional information of the count to the plurality of positions.

5. The material evaluation device according to claim 4, wherein
the processing unit
divides the material into a plurality of areas based on transition of the value of the count with respect to the plurality of positions, of the generated two-dimensional information of the count to the plurality of positions, and
calculates the physical property value of the material for each of the plurality of divided areas.

6. The material evaluation device according to claim 5, wherein
the processing unit
determines an order of the plurality of divided areas based on the value of the count, of the two-dimensional information of the count to the plurality of positions, and
calculates the physical property value of the material for each of the plurality of divided areas according to the determined order.

7. The material evaluation device according to claim 6, wherein
the processing unit calculates a linear physical property value among physical property values of the material, in a first area in the order among the plurality of divided areas, and calculates a nonlinear physical property value among the physical property values of the material, in a second or subsequent area in the order.

8. The material evaluation device according to any one of claims 1 to 7, wherein
the material includes a plurality of raw materials, and
the processing unit calculates a physical property value of the raw material for each of the plurality of raw materials when calculating the physical property value of the material.

9. A material evaluation method executed by a computer, the material evaluation method comprising:
storing N (N is an integer equal to or greater than 2) hysteresis curves that each represent a change in a second physical quantity of each time with respect to a change in a first physical quantity of N times at at least one of a plurality of positions of a material; and
refering to the storage unit, scan each of the N hysteresis curves with a value of the predetermined second physical quantity, for at least one of the plurality of positions, to extract a point, and one-dimensionally array the extracted points to generate one-dimensional information regarding the second physical quantity, and
calculating a physical property value of the material using the generated one-dimensional information regarding the second physical quantity.

10. A material evaluation program that causes at least one computer to execute a process, the process comprising:
storing N (N is an integer equal to or greater than 2) hysteresis curves that each represent a change in a second physical quantity of each time with respect to a change in a first physical quantity of N times at at least one of a plurality of positions of a material; and
refering to the storage unit, scan each of the N hysteresis curves with a value of the predetermined second physical quantity, for at least one of the plurality of positions, to extract a point, and one-dimensionally array the extracted points to generate one-dimensional information regarding the second physical quantity, and
calculating a physical property value of the material using the generated one-dimensional information regarding the second physical quantity.
